(19) Europäisches Patentamt
European Patent Office
Office européen des brevets

(11) EP 2 810 648 A1

(12) **EUROPEAN PATENT APPLICATION**

(43) Date of publication:
**10.12.2014 Bulletin 2014/50**

(21) Application number: **13170412.4**

(22) Date of filing: **04.06.2013**

(51) Int Cl.:
***A61K 31/519*** (2006.01) ***G01N 33/533*** (2006.01)
***G01N 33/68*** (2006.01) ***C07D 239/94*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Rauh, Daniel**
**67549 Worms (DE)**

(72) Inventors:
- **Rauh, Daniel**
  **67549 Worms (DE)**
- **Phan, Trang**
  **Ho Chi Minh City (VN)**
- **Nguyen, Hoang**
  **Ho Chi Minh City (VN)**
- **Fang, Zhizhou**
  **67112 Mutterstadt (DE)**
- **Simard, Jeffrey Raymond**
  **Salem, MA 01970 (US)**

(74) Representative: **Vossius & Partner**
**Siebertstrasse 4**
**81675 München (DE)**

(54) **Targeting domain-domain interaction for the identification of kinase modulators**

(57) The present invention relates to a polypeptide comprising or consisting of a protein kinase domain (KD) and one or more second domain(s), said second domain (s) binding to said KD and thereby modulating the activity of said KD; said polypeptide containing environmentally sensitive labels. The invention is also related to screening methods using said polypeptide, to heterocyclic inhibitors identified thereby, and the pharmaceutial use of said identified inhibitors.

EP 2 810 648 A1

SEQUENCE LISTING

<110> Rauh, Daniel

<120> Targeting domain-domain interaction for the identification of kinase modulators

<130> W1505 EP

<160> 1

<170> BiSSAP 1.2

<210> 1
<211> 480
<212> PRT
<213> Homo sapiens

<400> 1
Met Ser Asp Val Ala Ile Val Lys Glu Gly Trp Leu His Lys Arg Gly
1               5                   10                  15
Glu Tyr Ile Lys Thr Trp Arg Pro Arg Tyr Phe Leu Leu Lys Asn Asp
            20                  25                  30
Gly Thr Phe Ile Gly Tyr Lys Glu Arg Pro Gln Asp Val Asp Gln Arg
        35                  40                  45
Glu Ala Pro Leu Asn Asn Phe Ser Val Ala Gln Cys Gln Leu Met Lys
    50                  55                  60
Thr Glu Arg Pro Arg Pro Asn Thr Phe Ile Ile Arg Cys Leu Gln Trp
65                  70                  75                  80
Thr Thr Val Ile Glu Arg Thr Phe His Val Glu Thr Pro Glu Glu Arg
                85                  90                  95
Glu Glu Trp Thr Thr Ala Ile Gln Thr Val Ala Asp Gly Leu Lys Lys
            100                 105                 110
Gln Glu Glu Glu Glu Met Asp Phe Arg Ser Gly Ser Pro Ser Asp Asn
        115                 120                 125
Ser Gly Ala Glu Glu Met Glu Val Ser Leu Ala Lys Pro Lys His Arg
    130                 135                 140
Val Thr Met Asn Glu Phe Glu Tyr Leu Lys Leu Leu Gly Lys Gly Thr
145                 150                 155                 160
Phe Gly Lys Val Ile Leu Val Lys Glu Lys Ala Thr Gly Arg Tyr Tyr
                165                 170                 175
Ala Met Lys Ile Leu Lys Lys Glu Val Ile Val Ala Lys Asp Glu Val
            180                 185                 190
Ala His Thr Leu Thr Glu Asn Arg Val Leu Gln Asn Ser Arg His Pro
        195                 200                 205
Phe Leu Thr Ala Leu Lys Tyr Ser Phe Gln Thr His Asp Arg Leu Cys
    210                 215                 220
Phe Val Met Glu Tyr Ala Asn Gly Gly Glu Leu Phe Phe His Leu Ser
225                 230                 235                 240
Arg Glu Arg Val Phe Ser Glu Asp Arg Ala Arg Phe Tyr Gly Ala Glu
                245                 250                 255
Ile Val Ser Ala Leu Asp Tyr Leu His Ser Glu Lys Asn Val Val Tyr
            260                 265                 270
Arg Asp Leu Lys Leu Glu Asn Leu Met Leu Asp Lys Asp Gly His Ile
        275                 280                 285
Lys Ile Thr Asp Phe Gly Leu Cys Lys Glu Gly Ile Lys Asp Gly Ala
    290                 295                 300
Thr Met Lys Thr Phe Cys Gly Thr Pro Glu Tyr Leu Ala Pro Glu Val
305                 310                 315                 320
Leu Glu Asp Asn Asp Tyr Gly Arg Ala Val Asp Trp Trp Gly Leu Gly
                325                 330                 335
Val Val Met Tyr Glu Met Met Cys Gly Arg Leu Pro Phe Tyr Asn Gln

```
                340                 345                 350
Asp His Glu Lys Leu Phe Glu Leu Ile Leu Met Glu Glu Ile Arg Phe
            355                 360                 365
Pro Arg Thr Leu Gly Pro Glu Ala Lys Ser Leu Leu Ser Gly Leu Leu
    370                 375                 380
Lys Lys Asp Pro Lys Gln Arg Leu Gly Gly Gly Ser Glu Asp Ala Lys
385                 390                 395                 400
Glu Ile Met Gln His Arg Phe Phe Ala Gly Ile Val Trp Gln His Val
                405                 410                 415
Tyr Glu Lys Lys Leu Ser Pro Pro Phe Lys Pro Gln Val Thr Ser Glu
            420                 425                 430
Thr Asp Thr Arg Tyr Phe Asp Glu Glu Phe Thr Ala Gln Met Ile Thr
        435                 440                 445
Ile Thr Pro Pro Asp Gln Asp Asp Ser Met Glu Cys Val Asp Ser Glu
    450                 455                 460
Arg Arg Pro His Phe Pro Gln Phe Ser Tyr Ser Ala Ser Gly Thr Ala
465                 470                 475                 480
```

## Description

The present invention relates to a polypeptide comprising or consisting of a protein kinase domain (KD) and one or more second domain(s), said second domain(s) binding to said KD and thereby modulating the activity of said KD, and/or said second domain(s) being selected from (a) a pleckstrin homology domain (PH domain), said KD in that case being selected from Akt, PDK1, PKC$\mu$, BTK, ITK, GRK2, Tec, ETK, TXK and ROCK; (b) an SH2 domain, said KD in that case being selected from Abl, Src, BLK, BMX, BTK, Fer, Fes, FRK, FYN, HCK, JAK2, ITK, Lck, Lyn, MATK, BRK, Syk, Tec and TXK; (c) an SH3 domain, said KD in that case being selected from Abl, Src, RSK and Lck; (d) a protein kinase autoinhibition domain, said KD in that case being selected from AMPK, PAK1 and PKG; (e) a protein kinase hydrophobic motif, said KD in that case being selected from Akt, PDK1, PKA and S6K; (f) a C1 domain, said KD in that case being selected from PKC (including isoforms $\alpha$, $\beta_I$, $\beta_{II}$, $\gamma$, $\delta$, $\varepsilon$, $\eta$, $\theta$, |, , $\lambda$), ARAF, BRAF, MRCK, CIT, ROCK1 and ROCK2; and (g) a C2 domain, said KD in that case being selected from PKC$\alpha$, PKC$\beta_I$, PKC$\beta_{II}$ and PKC$\gamma$; said polypeptide containing, (1) if one second domain is present, within a distance of not more than 15 Å and not less than 1 Å from the interface between said KD and said second domain, exactly one or exactly two environmentally sensitive labels, or, (2) if more than one second domain is present, within a distance of not more than 15 Å and not less than 1 Å from each of one, more or all of the interfaces between said KD and said second domains, respectively, exactly one or exactly two environmentally sensitive labels, wherein said label(s) is/are (a) fluorophore(s), (a) spin label(s) or (an) isotope label(s), (i) the fluorophore or said spin label is covalently bound to a thiol group or side chain amino group of an amino acid residue, (ii) the isotope label is a moiety comprising one or more atoms which are isotopes with non-zero nuclear spin, said moiety being covalently bound to a thiol group or side chain amino group of an amino acid residue, or the isotope label is an amino acid residue, said amino acid residue comprising one or more atoms which are isotopes with non-zero nuclear spin, wherein said isotopes with non-zero nuclear spin are enriched as compared to the natural occurrence thereof, wherein, in case exactly two labels are present for a given interface, a first label is present on said KD and a second label is present on the second domain forming said given interface.

In this specification, a number of documents including patent applications and manufacturer's manuals are cited. The disclosure of these documents, while not considered relevant for the patentability of this invention, is herewith incorporated by reference in its entirety. More specifically, all reference documents are incorporated by reference to the same extent as if each individual document was specifically and individually indicated to be incorporated by reference.

Two decades of extensive kinase research have resulted in a substantial number of kinase inhibitors of various types (Rabiller, M. et al. Proteus in the world of proteins: conformational changes in protein kinases. Arch. Pharm. (Weinheim, Ger.) 343, 193-206, doi:10.1002/ardp.201000028 (2010)). The compounds that have been developed over the last years have found great application as probes to dissect and understand kinase function in the context of signaling networks, as well as therapeutics for the treatment of diseases such as cancer, arthritis, autoimmune disorders, allergies or diabetes (Bhagwat, S.S. Kinase inhibitors for the treatment of inflammatory and autoimmune disorders. Purinergic Signal. 5, 107-115, doi:10.1007/s11302-008-9117-z (2009)). The majority of these inhibitors target the enzymatic activity of the respective kinase. However, recent studies clearly showed that kinase activity is much more complex than their pure phosphorylation capacity (Rauch, J., Volinsky, N., Romano, D. & Kolch, W. The secret life of kinases: functions beyond catalysis. Cell Commun. Signal. 9, 23 (2011)). In fact, it has been demonstrated that kinases possess functions beyond their enzymatic activity, e.g. as scaffolding proteins to form complexes or by competing for protein-protein interactions. Modulators of such interactions have various advantages over classic antagonistic inhibitors when it comes to the pharmacological modulation of disease states, thereby providing new strategies for drug development. Inhibitors that target these interactions with an allosteric mode of action have great value and higher intrinsic selectivity, as these interactions are often less conserved than the ATP-binding site of the protein kinase domain.

In view of the above described advantages of kinase modulators which do not bind to the most conserved regions of the kinase domain such as the ATP-binding pocket, there is a need for means and methods to identify such kinase modulators. The solution to this technical problem is provided by the embodiments characterized in the claims.

Accordingly, the present invention in a first aspect, provides a polypeptide comprising or consisting of a protein kinase domain (KD) and one or more second domain(s), said second domain(s) binding to said KD and thereby modulating the activity of said KD, and/or said second domain(s) being selected from (a) a pleckstrin homology domain (PH domain), said KD in that case being selected from Akt, PDK1, PKC$\mu$, BTK, ITK, GRK2, Tec, ETK, TXK and ROCK; (b) an SH2 domain, said KD in that case being selected from Abl, Src, BLK, BMX, BTK, Fer, Fes, FRK, FYN, HCK, JAK2, ITK, Lck, Lyn, MATK, BRK, Syk, Tec and TXK; (c) an SH3 domain, said KD in that case being selected from Abl, Src, RSK and Lck; (d) a protein kinase autoinhibition domain, said KD in that case being selected from AMPK, PAK1 and PKG; (e) a protein kinase hydrophobic motif, said KD in that case being selected from Akt, PDK1, PKA and S6K; (f) a C1 domain, said KD in that case being selected from PKC (including isoforms $\alpha$, $\beta_I$, $\beta_{II}$, $\gamma$, $\delta$, $\varepsilon$, $\eta$, $\theta$, |, $\zeta$, $\lambda$), ARAF, BRAF, MRCK, CIT, ROCK1 and ROCK2; and (g) a C2 domain, said KD in that case being selected from PKC$\alpha$, PKC$\beta_I$, PKC$\beta_{II}$ and PKC$\gamma$; said polypeptide containing, (1) if one second domain is present, within a distance of not more than 15 Å and not less than 1 Å from the interface between said KD and said second domain, exactly one or exactly two environmentally

sensitive labels, or, (2) if more than one second domain is present, within a distance of not more than 15 Å and not less than 1 Å from each of one, more or all of the interfaces between said KD and said second domains, respectively, exactly one or exactly two environmentally sensitive labels, wherein said label(s) is/are (a) fluorophore(s), (a) spin label(s) or (an) isotope label(s), (i) the fluorophore or said spin label is covalently bound to a thiol group or side chain amino group of an amino acid residue, (ii) the isotope label is a moiety comprising one or more atoms which are isotopes with non-zero nuclear spin, said moiety being covalently bound to a thiol group or side chain amino group of an amino acid residue, or the isotope label is an amino acid residue, said amino acid residue comprising one or more atoms which are isotopes with non-zero nuclear spin, wherein said isotopes with non-zero nuclear spin are enriched as compared to the natural occurrence thereof, wherein, in case exactly two labels are present for a given interface, a first label is present on said KD and a second label is present on the second domain forming said given interface.

This includes a polypeptide comprising or consisting of a protein kinase domain (KD) and a second domain, said second domain binding to said KD and thereby modulating the activity of said KD, and/or said second domain being selected from (a) a pleckstrin homology domain (PH domain), said KD in that case being selected from Akt, PDK1, PKC$\mu$, BTK, ITK, GRK2, Tec, ETK, TXK and ROCK; (b) an SH2 domain, said KD in that case being selected from Abl, Src, BLK, BMX, BTK, Fer, Fes, FRK, FYN, HCK, JAK2, ITK, Lck, Lyn, MATK, BRK, Syk, Tec and TXK; (c) an SH3 domain, said KD in that case being selected from Abl, Src, RSK and Lck; (d) a protein kinase autoinhibition domain, said KD in that case being selected from AMPK, PAK1 and PKG;(e) a protein kinase hydrophobic motif, said KD in that case being selected from Akt, PDK1, PKA and S6K; (f) a C1 domain, said KD in that case being selected from PKC (including isoforms $\alpha$, $\beta_I$, $\beta_{II}$, $\gamma$, $\delta$, $\varepsilon$, $\eta$, $\theta$, I, $\zeta$, $\lambda$), ARAF, BRAF, MRCK, CIT, ROCK1 and ROCK2; and (g) a C2 domain, said KD in that case being selected from PKC$\alpha$, PKC$\beta_I$, PKC$\beta_{II}$ and PKC$\gamma$; said polypeptide containing, within a distance of not more than 15 Å and not less than 1 Å from the interface between said KD and said second domain, exactly one or exactly two environmentally sensitive labels, wherein said label(s) is/are (a) fluorophore(s), (a) spin label(s) or (an) isotope label(s), (i) the fluorophore or said spin label is covalently bound to a thiol group or side chain amino group of an amino acid residue, (ii) the isotope label is a moiety comprising one or more atoms which are isotopes with non-zero nuclear spin, said moiety being covalently bound to a thiol group or side chain amino group of an amino acid residue, or the isotope label is an amino acid residue, said amino acid residue comprising one or more atoms which are isotopes with non-zero nuclear spin, wherein said isotopes with non-zero nuclear spin are enriched as compared to the natural occurrence thereof, wherein, in case exactly two labels are present, a first label is present on said KD and a second label is present on said second domain.

The term "kinase" is well-known in the art and refers to a type of enzyme that transfers phosphate groups from high-energy donor molecules, such as ATP, to specific target molecules such as proteins. Kinases are classified under the enzyme commission (EC) number 2.7. Kinases which phosphorylate proteins are also referred to as protein kinases. Kinases according to the present invention are protein kinases. According to substrate specificity, protein kinases can be subdivided into serine/threonine kinases (EC 2.7.11, e.g. p38$\alpha$), tyrosine kinases (EC 2.7.10, e.g. the EGFR kinase domain), histidine kinases (EC 2.7.13), aspartic acid/glutamic acid kinases and mixed kinases (EC 2.7.12) which have more than one specificity (e.g. MEK being specific for serine/threonine and tyrosine).

Amino acid sequences, secondary and tertiary structure of protein kinases contain a plurality of conserved elements which are well-known in the art; see, for example, Hanks, Steven K., Hunter, Tony. The eukaryotic protein kinase superfamily: kinase (catalytic) domain structure and classification, Academic Press London 1995, 9, 576-596; Rabiller, M., Getlik, M., Klüter, S., Richters, A., Tückmantel, S., Simard, J.R., Rauh, D. Proteus in the world of proteins: Conformational changes in protein kinases, Arch. Pharm. Chem. Life Sci. 2010, 343, 193-206; and Serota Taylor, S., Radzio-Andzelm, Elzbieta, Three protein kinase structures define a common motif. Structure 1994, 2:345-355. All protein kinases comprise or consist of at least a protein kinase domain which is an autonomous folding unit conferring the capability to phosphorylate a given target. The occurrence of a protein kinase domain within a given polypeptide as well as its boundaries in the amino acid sequence of said polypeptide may be determined by experimental or bioinformatics means. As regards experimental means, truncation experiments may be performed and the capability of the obtained truncated forms to phosphorylate a given target may be assessed. Truncated forms, herein also referred to as fragments, which maintain the capability to phosphorylate a given target are those which comprise a protein kinase domain. As regards bioinformatic means, several consensus sequences and statistical models of protein kinase domains are available. A preferred statistical model is the hidden mark of model (HMM) as comprised in the Pfam database (The Pfam protein families database: M. Punta, P.C. Coggill, R.Y. Eberhardt, J. Mistry, J. Tate, C. Boursnell, N. Pang, K. Forslund, G. Ceric, J. Clements, A. Heger, L. Holm, E.L.L. Sonnhammer, S.R. Eddy, A. Bateman, R.D. Finn; Nucleic Acids Research (2012) Database Issue 40: D290-D301). The protein kinase domain HMM of the Pfam database is designated "pkinase". A preferred version of the Pfam database is version 27.0 of March 2013.

The term "second domain" refers to a second autonomous folding unit comprised in said polypeptide. As is known in the art, many protein kinases comprise, in addition to a protein kinase domain, one or more further domains. A subset of these further domains is known to bind to said protein kinase domain and thereby modulate the activity, preferably catalytic activity thereof. These latter features are minimal features characterizing a second domain in accordance with the present invention. Whether any given domain meets the requirements of the second domain in accordance with the present invention may be determined by binding assays which are known in the art including a Biacore assay. Whether any modulatory effect, preferably inhibitory effect goes along with the binding of said second domain to said protein kinase domain may be determined by known kinase assay which determine and quantify the catalytic activity of a protein kinase domain. A modulatory effect is a change and an inhibitory effect is a decrease in kinase catalytic activity by at least 10%, preferably 20%, 30%, 40%, 50%, 60%, 70%, 80%, 90%, 95%, 96%, 97%, 98% or 99%. To the extent the term "modulatory" extends to activating effects, it is understood that twofold, threefold, fourfold, fivefold, tenfold or higher degrees of activation fall under the term "modulatory effect".

As noted above, the term "second domain" is connected to its regulatory effect on the kinase domain. Generally speaking, wild-type kinases and also polypeptides according to the present invention may comprise three distinct types of domains, namely (i) a kinase domain, (ii) one or more second, i.e. regulatory domains, and (iii) one or more further domains, said further domains having no regulatory effect on the kinase domain. As regards the polypeptide according to the present invention, preference is given to the presence of exactly two domains, namely a kinase domain and exactly one second domain. Having said that, a number of alternative domain architectures are envisaged as well, including polypeptides with one kinase domain and two second domains, polypeptides with a kinase domain, one second domain and one further domain, polypeptides with a kinase domain, one second domain and two or more further domains, polypeptides with two or more second domains and one or more further domains, etc.

The term "polypeptide" preferably has its meaning as established in the art and relates to a polycondensate of alpha amino acids, preferably of the twenty naturally occurring proteinogenic alpha amino acids. In its widest sense, the term "polypeptide" as used herein includes molecules comprising said protein kinase domain and said second domain, whereby these two domains are connected by a linker of which said linker is not necessarily peptidic in nature. Suitable non-peptidic linkers include polymethylene or PEG linkers. Preferably, the total number of atoms (disregarding hydrogens) forming such a linker is below 50, 40, 30, 20 or 10 atoms. To the extent the term "polypeptide" refers to a polycondensate of alpha amino acids without any non-peptidic linker, it is understood that the protein kinase domain and the second domain may be linked by that sequence of amino acids which connects these two domains in the wild type counterpart, i.e., that naturally occurring kinase which comprises the specific protein kinase domain and second domain occurring in a given polypeptide according to the present invention. Alternatively, a different peptidic linker may be used in place of the naturally occurring linker. Examples of non-naturally occurring peptidic linkers include oligoglycine stretches.

To the extent polypeptides comprise at least one protein kinase domain, such polypeptides are also referred to as "kinases" herein. Preferred kinases are those which comprise at least one second domain as defined herein above.

The second domains in accordance with items (a) to (g) of the first aspect as such are known in the art and described in numerous references.

More specifically, the PH domain is known in the art and described, for example, in R. J. Haslam, H. B. Koide & B. A. Hemmings. Pleckstrin domain homology. Nature 1993 363, 309-310.

SH3 and SH2 domains are described, for example, in T. Pawson. SH2 and SH3 domains. Curr. Opin. Struct. Biol., 1992, 2, 432-437; T. Pawson, J. Schlessingert. SH2 and SH3 domains. Current Biology, 1993, 3, 434-442; B. J. Mayer, D. Baltimore. Signalling through SH2 and SH3 domains. Trends Cell Biol, 1993, 3, 8-13; and T. E. Smithgall. SH2 and SH3 Domains: Potential Targets for Anti-Cancer Drug Design. J. Pharmacol. Toxicol. Methods, 1995, 34, 125-132.

The term "autoinhibition domain" refers to domains which are diverse in terms of their sequences. The term was coined phenomenologically based on its function. Description of the autoinhibition domain of AMPK can be found, for example, in L. Chen, Z.-H. Jiao, L.-S. Zheng, Y.-Y. Zhang, S.-T. Xie, Z.-X. Wang, J.-W. Wu. Structural insight into the autoinhibition mechanism of AMP-activated protein kinase. 2009, 459, 1146-1150; and T. Pang, B. Xiong, J.-Y. Li, B.-Y. Qiu, G.-Z. Jin, J.-K. Shen, J. Li. Conserved a-Helix Acts as Autoinhibitory Sequence in AMP-activated Protein Kinase a Subunits. J. Biol. Chem., 2007, 282, 495-506.

Description of the autoinhibition domain of PAK1 can be found, for example, in M. Lei, W. Lu, W. Meng, M.-C. Parrini, M. J. Eck, B. J. Mayer, S. C. Harrison. Structure of PAK1 in an Autoinhibited Conformation Reveals a Multistage Activation Switch. Cell, 2000, 102, 387-397. G. M. Bokoch, Biology of the p21-activated Kinases. Annu. Rev. Biochem., 2003, 72, 743-781. Z. M. Jaffer, J. Chernoff, p21-Activated kinases: three more join the Pak. Int. J. Biochem. Cell Biol., 2002, 34, 713-717; M. Carla Parrini, M. Lei, S. C. Harrison, B. J. Mayer. Pak1 Kinase Homodimers Are Autoinhibited in trans and Dissociated upon Activation by Cdc42 and Rac1. Mol. Cell, 2002, 9, 73-83; and Y.-H. Hsu, D. A. Johnson, J. A. Traugh. Analysis of Conformational Changes during Activation of Protein Kinase Pak2 by Amide Hydrogen/Deuterium Exchange. J. Biol. Chem., 2008, 283, 36397-36405.

Description of the autoinhibition domain of PKG can be found, for example, in S. H. Francis, J. D. Corbin. Structure and Function of Cyclic Nucleotide-dependent Protein Kinases. Annu. Rev. Physiol., 1994, 56, 237-272; F. Hofmann, D. Bernhard, R. Lukowski, P. Weinmeister. cGMP regulated protein kinases (cGK). Handb. Exp. Pharmacol., 2009, 191, 137-162; B. W. Osborne, J. Wu, C. J. McFarland, C. K. Nickl, B. Sankaran, D. E. Casteel, V. L. Woods, Jr., A. P. Kornev, S. S. Taylor, W. R. Dostmann. Crystal Structure of cGMP-Dependent Protein Kinase Reveals Novel Site

of Interchain Communication. Structure, 2011, 19, 1317-1327; and R. A. Atkinson, V. Saudek, J. P. Huggins, J. T. Pelton. 'H NMR and Circular Dichroism Studies of the N-Terminal Domain of Cyclic GMP Dependent Protein Kinase: A Leucine/isoleucine Zipper. Biochemistry, 1991, 30, 9387-9395.

The hydrophobic motif is described in N. Kannan, N. Haste, S. Taylor, A. F. Neuwald. The hallmark of AGC kinase functional divergence is its C-terminal tail, a cis-acting regulatory module. Proc. Natl. Acad. Sci., 2007, 104, 1272-1277; and A. C. Newton, Regulation of the ABC kinases by phosphorylation: protein kinase C as a paradigm. Biochem. J., 2003, 370, 361-371.

Exemplary description of the C1 domain can be found in F. Colón-González, M. G. Kazanietz. C1 domains exposed: From diacylglycerol binding to protein-protein interactions. Biochim. Biophys. Acta, 2006, 1761, 827-837; G. Zhang, M. G Kazanietz, P. M. Blumberg, J. H Hurley. Crystal structure of the Cys2 activator-binding domain of protein kinase C$\delta$ in complex with phorbol ester. Cell, 1995, 81, 917-924; and A. Azzi, D. Boscoboinik, C. Hensey. The protein kinase C family. Eur. J. Biochem., 1992, 208, 547-557.

The C2 domain is described, for example, in R. B. Sutton, B. A Davletov, A. M. Berghuis, T. C. Sudhof, S. R Sprang. Structure of the first C2 domain of synaptotagmin I: A novel Ca2+/phospholipid-binding fold. Cell, 1995, 80, 929-938; and D. Zhang, L. Aravind. Identification of novel families and classification of the C2 domain superfamily elucidate the origin and evolution of membrane targeting activities in eukaryotes. Gene, 2010, 469, 18-30.

As noted above, the term "modulating" includes "inhibiting" and "activating". With regard to the specific second domains in accordance with items (a) to (g) it is known in the art which effect is exerted by said second domain. For example, the pleckstrin homology domain, in particular the pleckstrin homology domain as comprised in kinases of the Akt family, is known to inhibit the activity of the kinase domain when said pleckstrin homology domain is bound to said kinase domain. As regards SH2 and SH3 domains, in particular in Abl, the kinase domain is inactive in a bound state wherein the ATP binding site is blocked. The hydrophobic motif, in particular in PDK1, when bound to the kinase domain, has an activating effect. The C1 domain, in particular in PKC isoforms $\alpha$, $\beta_I$, $\beta_{II}$ and $\gamma$, is characterized in that the bound form (C1 bound to KD) is inactive.

For each of the mentioned types of interaction between kinase domain and second domain, i.e. activating and inhibiting, a compound, preferably small organic molecule, which may be identified by using the polypeptides and methods in accordance with the present invention, may have an inhibitory or activating effect on the kinase domain. To explain further, such compound may stabilize a closed conformation, i.e. the bound state of kinase domain and second domain, namely by contacting both domains and holding them together. If the bound state between kinase domain and second domain is such that activity of the kinase domain is inhibited, such compound will be an inhibitor of the kinase domain. On the other hand, if the closed conformation is characterized by an activated state of the kinase domain, such compound will have an activating effect on the kinase domain. Alternatively, such compound may be chosen such that it prevents formation of the closed conformation, or interferes with the interdomain interactions that are needed for closing, then the regulatory effect exerted by the second domain on the kinase domain will be suppressed. More specifically, in case the second domain has an inhibitory effect on the activity of the kinase domain, such compound will maintain the kinase domain in an activated state. On the other hand, if the second domain exerts an activating effect on the kinase domain, such compound will prevent such activating effect and the kinase domain, in the presence of said compound, will stay in its lower activity or inactive state.

Generally speaking, preference will be given to compounds which have an inhibitory effect on the activity of the kinase domain. In view of the above, it is understood that two routes are made available for the identification of such inhibitors by the present invention. First, an inhibitory interaction between a kinase domain and a second domain may be reinforced by such compound, or an activating interaction between kinase domain and second domain may be prevented by a compound identifiable with the means and methods according to the present invention.

The common names of kinases as used herein are well-established in the art. Further information on the respective kinase can be found, for example, in the UniProt database (The UniProt Consortium. Ongoing and future developments at the Universal Protein Resource. Nucleic Acids Res. 39, D214-D219 (2011)), preferably in UniProt release 2013_06 of May 21, 2013.

The term "label" is well-known in the art. In the present case, the label according to invention is to be selected from a fluorophore, a spin label and an isotope label which are discussed in more detail below. Importantly, the label according to the invention is a moiety which does not occur in the wild type form of said kinase. For example, a fluorophore as comprised in a naturally occurring amino acid is not a label according to the invention. Similarly, an amino acid containing a $^{13}C$ nucleotide is not a label according to the invention if the frequency of $^{13}C$ at the given position merely arises from the natural distribution of isotopes.

In order to respond to any change in the interaction between said protein kinase domain and said second domain, such change preferably being the binding of said second domain to said protein kinase domain, the label has to be in the proximity of the interface between the two domains. Such proximity is defined in terms of concrete distances. More specifically, the label has to be between 1 and 15 Å from the interface between the two domains. The label is an environmentally sensitive label, i.e., the label changes its spectral characteristics in response to said binding. Depending

on the type of label, said spectral characteristic is selected from fluorescence emission signal, EPR signal and NMR signal. As such, it is understood that exactly one environmentally sensitive label within 1 to 15 Å from said interface is sufficient to serve as a readout for any binding between the protein kinase domain and second domain.

In an alternative setup, though, two environmentally sensitive labels may be placed within said zone ranging from 1 Å to 15 Å from said interface. In that case, exactly one label is present within 1 and 15 Å from the interface on said protein kinase domain, and exactly one label is present on said second domain within 1 and 15 Å from said interface. In such a setup, one of the two labels, also referred to as first label, may emit a signal, wherein the respective other label, also referred to as second label, would act as a quencher, quenching being more efficient in case of closer spatial proximity. Also envisaged is that the first label is weakly flourecent and transmits energy to a second label and this transfer increases as the labels move closer together.

It is understood that outside said region from 1 to 15 Å on either domain, further labels, possibly but not necessarily including environmentally sensitive labels, may be present. In preferred embodiments, though, exactly one or exactly two labels are present in the entire polypeptide, said one or said two labels being environmentally sensitive labels and being located within 1 to 15 Å from said interface or within any of the preferred distance ranges specified below.

Preferred upper limits for the distance from said interface include 14, 13, 12, 11, 10, 9, 8, 7, 6 and 5 Å. Preferred lower limits for the distance from said interface include 2, 3, 4, 5, 6, 7, 8, 9 and 10 Å. It is understood that any label is placed such that it does not interfere with binding events at the interface. Within these preferred ranges, the skilled person can choose specific distances based on the following rule: closer to the interface means higher sensitivity but greater probability of interfering with the interaction of interest.

Generally speaking, the term "region", if not indicated otherwise, is used herein to designate the region from 1 to 15 Å (or any of the preferred sub-intervals thereof) from the interface, either on the protein kinase domain or the second domain.

The term "interface" as used herein refers to a subset of amino acids within said polypeptide. More specifically, it refers to that specific subset which is involved in the formation of contacts between said protein kinase domain and said second domain upon binding of said protein kinase domain and said second domain. Whether or not a given amino acid is part of said interface is determined using the three-dimensional structure of the bound state of said protein kinase domain and said second domain. Such three-dimensional structure may be determined using X-ray crystallography, NMR spectroscopy or molecular modelling. Preference is given to three-dimensional structures obtained by X-ray crystallography, followed by three-dimensional structures determined by NMR. If in said three-dimensional structure the atom of any amino acid of said protein kinase domain is within less than 3.5 Å of any atom of any amino acid of said second domain, then said amino acid of said protein kinase domain as a whole as well as said amino acid of said second domain as a whole are part of said interface.

Distances between labels and said interface are defined as follows. If the label is a fluorophore, a spin label or an isotope label, the isotope label being a moiety comprising one or more atoms which are isotopes with non-zero nuclear spin, said moiety being covalently bound to a thiol group or side chain amino group of an amino acid residue, then the distance between the sulfur atom of said thiol group or the nitrogen atom of said amino group on the one hand and the closest C-alpha atom of an amino acid of said interface on the other hand is determined. In case said isotope label is an amino acid residue, the distance between the C-alpha atom of said amino acid residue on the one hand and the C-alpha atom of an amino acid of said interface on the other hand is determined.

To give an example, the interface between the protein kinase domain and the PH domain in the Akt kinase may be defined by reference to the 3D structure of pdb entries 3096 and 4EJN as follows: residues 14-19, 23, 25, 36-39, 50-55, 59, 61, 63, 76-85 on the PH domain and residues 161, 179, 181, 185-188, 202-206, 210-213, 223, 225, 227, 265, 268-273, 292, 294-297, 309, 319-328, 358, 362 on the kinase domain. Residue numbering refers to that of SEQ ID NO: 1.

The terms "wild type form" or "wild type" as used further below are meant to distinguish naturally occurring polypeptides from (i) polypeptides which have sequences that are modified in accordance with the present invention for the purpose of labeling and (ii) polypeptides containing a label as defined in the first aspect. Accordingly, the term "wild type form" includes naturally occurring mutants such as, for example, disease-relevant mutants, provided that those mutants are neither labeled in accordance with the invention nor have a sequence which is modified for the purpose of labeling. It is understood that in such naturally occurring mutant forms the mutated position is generally irrelevant for and not useful for labeling.

In other words, a given polypeptide or kinase may occur in at least three different forms. First, there is the wild type form of the kinase which, for the purpose of labeling, might require certain modifications of the sequence such as introduction of a site amenable to labeling. Such modifications are further detailed below. Another type of modification which might be necessary is the removal of one or more sites where labeling is not desired. In either of the two cases, a difference in sequence will arise between the wild type form of the kinase and the kinase which is amenable to labeling. Upon labeling, which may be achieved by reacting with reactive forms of fluorophores, spin labels and isotope labels, a further form is obtained which is the polypeptide according to the invention. Said polypeptide according to the invention

differs from the wild type form thereof in that it is labeled, and, if the sequence of the wild type form was not suitable for labeling, by one or more sequence modifications. Further differences from said wild type form may arise in that a polypeptide according to the present invention may be a subsequence of said wild type form. To give an example, if said second domain is a PH domain, a naturally occurring kinase may comprise a protein kinase domain, a PH domain and a further domain. Yet, a subsequence comprising said protein kinase domain and said PH domain as the only two domains would qualify as a polypeptide in accordance with the present invention. Having said that, it is also envisaged to use the complete sequences of the wild type form for the purpose of generating a polypeptide in accordance with the present invention, by which said polypeptide is modified only by introduction of the label and, to the extent necessary, modifying the sequence by introducing a site amenable to labeling and/or removing or mutating sites which shall not be labelled.

The term "fluorophore" is well-known in the art and denotes a molecule or functional group within a molecule which absorbs energy at a specific wavelength and emits energy, i.e. light at a different (but equally specific) wavelength immediately upon absorbance (unlike the case in phosphorescence) without the involvement of a chemical reaction (as the case in bioluminescence). This type of emission is known as fluorescence. Usually the wavelength of the absorbed photon is in the ultraviolet range but can reach also into the visible and infrared range. The wavelength of the emitted light is shifted towards longer wavelengths as compared to the absorption wavelength and is usually in the visible range. The amount and wavelength of the emitted energy depend primarily on the properties of the fluorophore but is also influenced by the chemical environment surrounding the fluorophore. Fluorophores can be sensitive to changes in their environment. Fluorophores according to the invention are sensitive to the environment. This includes changes in the polarity and/or charge which occurs when the conformation of the molecule to which they are attached changes. Fluorescence occurs when a molecule relaxes to its ground state after being electronically excited which, for commonly used fluorescent compounds that emit photons with energies from the UV to near infrared, typically happens in the range of between 0.5 and 20 nanoseconds. Fluorophores typically exhibit changes in intensity and/or a shift in the wavelength of maximum emission depending on the polarity of the surrounding chemical environment. In the present case, the chemical environment is formed by the structural elements of the kinase, in particular those structural elements of the protein kinase domain and the second domain, in particular the interface between the two domains, as well as by surrounding molecules including solvent molecules.

Preferred fluorophores are Acrylodan, PyMPO, DY-647, IANBD, IAEDANS, IAANS, Texas Red, Atto 565 and Eosin.

Other fluorophores which may be used are coumarin-based compounds, benzoxadiazole-based compounds, dapoxyl-based compounds, biocytin-based compounds, fluorescein, sulfonated rhodamine-based compounds such as AlexaFluor dyes (Molecular Probes), Atto fluorophores (Atto Technology) or Lucifer Yellow. Coumarin-based fluorophores are moderately sensitive to environment and 7-diethylamino-3-(4'-maleimidylphenyl)-4-methylcoumarin (CPM) is an example. Benzoxadiazole fluorophores are also commonly used for forming protein-fluorophore conjugates and have a strong environmental dependence with 7-fluorobenz-2-oxa-1,3-diazole-4-sulfonamide (ABD-F) and *N*-((2-(iodoacetoxy)ethyl)-*N*-methyl)amino-7-nitrobenz-2-oxa-1,3-diazole ester (IANBD) as examples. PyMPO maleimide (for thiols) or succinimide ester (for amines) and various other dapoxyl dyes have good absorptivity and exceptionally high environmental sensitivity. Examples are 1-(2-maleimidylethyl)-4-(5-(4-methoxyphenyl)oxazol-2-yl)pyridinium methanesulfonate (PyMPO-maleimide), 1-(3-(succinimidyloxycarbonyl)benzyl)-4-(5-(4-methoxyphenyl)oxazol-2-yl) pyridinium bromide (PyMPO-succinimidyl ester) and Dapoxyl (2-bromoacetamidoethyl) sulphonamide.

Recording the fluorescence emission signal at one or more wavelengths or a spectrum is usually accomplished using a fluorescence spectrometer or fluorimeter. Fluorescence spectroscopy or fluorimetry or spectrofluorimetry is a type of electromagnetic spectroscopy which analyzes fluorescence, or other emitted light, from a sample. It involves using a beam of light, usually ultraviolet light, that excites the electrons in certain molecules and causes them to emit light of a lower energy upon relaxation, typically, but not necessarily, visible light.

Two general types of instruments exist which can both be employed in the method of the invention: Filter fluorimeters use filters to isolate the incident light and fluorescent light, whereas spectrofluorimeters use diffraction grating monochromators to isolate the incident light and fluorescent light. Both types utilize the following scheme: The light from an excitation source passes through a filter or monochromator and strikes the sample. A proportion of the incident light is absorbed by the sample, and some of the molecules in the sample fluoresce. The fluorescent light is emitted in all directions. Some of this fluorescent light passes through a second filter or monochromator and reaches a detector, which is usually placed at 90° to the incident light beam to minimize the risk of transmitted or reflected incident light reaching the detector. Various light sources may be used as excitation sources, including lasers, photodiodes, and lamps; xenon and mercury vapor lamps in particular. The detector can either be single-channeled or multi-channeled. The single-channeled detector can only detect the intensity of one wavelength at a time, while the multi-channeled detects the intensity at all wavelengths simultaneously, making the emission monochromator or filter unnecessary. The different types of detectors have both advantages and disadvantages. The most versatile fluorimeters with dual monochromators and a continuous excitation light source can record both an excitation spectrum and a fluorescence spectrum.

When measuring fluorescence spectra, the wavelength of the excitation light is kept constant, preferably at a wavelength of high absorption, and the emission monochromator scans the spectrum. For measuring excitation spectra, the wavelength passing though the emission filter or monochromator is kept constant and the excitation monochromator is scanning. The excitation spectrum generally is identical to the absorption spectrum as the fluorescence intensity is proportional to the absorption (for reviews see Rendell, 1987; Sharma and Schulman,1999; Gauglitz and Vo-Dinh, 2003; Lakowicz, 1999).

The term "spin label" (SL) denotes a molecule, generally an organic molecule, which possesses an unpaired electron, usually on a nitrogen atom. Spin labels are used as tools for probing proteins using electron paramagnetic resonance (EPR) spectroscopy. The site-directed spin labeling (SDSL) technique allows one to monitor the conformation and dynamics of a protein. In such examinations, amino acid-specific SLs can be used. In a further preferred embodiment, the thiol-reactive spin-label is a nitroxide radical.

A preferred method for site-specifically labeling protein sequences with a spin-label is the reaction between methanethiosulfonate spin label and cysteine, to give the spin-labeled cysteine side chain, Cys-SL:

MeS(O)2SSR + R'SH ---> R'SSR + MeS(O)2SH

where R is the nitroxide group and R'SH is a protein with a cysteine sulfhydryl, and R'SSR is the spin-labeled protein. The cysteines for labeling are placed in the desired sequence position either through solid-phase techniques or through standard recombinant DNA techniques.

Site-directed spin labeling is a technique for investigating protein local dynamics using electron spin resonance. SDSL is based on the specific reaction of spin labels carrying a reactive group with amino acids. A spin label built in protein structures can be detected by EPR spectroscopy. In SDSL, sites for attachment of spin labels such as thiol or side chain amino groups, if not naturally present, are introduced into recombinantly expressed proteins, for example by site-directed mutagenesis. In other words, by the above techniques, spin labels can be introduced into a kinase such that said kinase is specifically labeled at, preferably only at the desired position. Reactive functional groups contained within the spin label determine their specificity. At neutral pH, protein thiol groups specifically react with functional groups such as methanethiosulfonate, maleimide and iodoacetamide, creating a covalent bond with the amino acid cysteine. It is understood that the spin label according to the main embodiment no longer contains a reactive functional group. As required by the invention, the spin label (already) is connected to an amino acid of the kinase via a covalent bond. Spin labels are unique molecular reporters in that they are paramagnetic, i.e. they contain an unpaired electron. For example, nitroxide spin labels are widely used for the study of macromolecular structure and dynamics because of their stability and simple EPR signal. The nitroxyl radical (N-O) is usually incorporated into a heterocyclic ring such as pyrrolidine, and the unpaired electron is predominantly localized to the N-O bond. Once incorporated into the protein, a spin label's motions are dictated by its local environment. Because spin labels are exquisitely sensitive to motion, this has profound effects on the EPR spectrum of the spin-label attached to the protein.

The signal arising from an unpaired electron can provide information about the motion, distance, and orientation of unpaired electrons in the sample with respect to each other and to the external magnetic field. For molecules free to move in solution, EPR works on a much faster time-scale than NMR (Nuclear Magnetic Resonance) spectroscopy, and accordingly can reveal details of much faster molecular motions, i.e. nanoseconds as opposed to microseconds for NMR. The gyromagnetic ratio of the electron is orders of magnitude larger than of nuclei commonly used in NMR, and so the technique is more sensitive than NMR.

Electron paramagnetic resonance (EPR) or electron spin resonance (ESR) spectroscopy is a technique for studying chemical species that have one or more unpaired electrons, such as organic and inorganic free radicals or inorganic complexes possessing a transition metal ion. The basic physical concepts of EPR are analogous to those of nuclear magnetic resonance (NMR), but it is electron spins that are excited instead of spins of atomic nuclei. Because most stable molecules have all their electrons paired, the EPR technique is less widely used than NMR. However, this limitation to paramagnetic species also means that the EPR technique is one of great specificity, since ordinary chemical solvents and matrices do not give rise to EPR spectra.

An isotope label according to the invention is, as defined above, a moiety comprising one or more atoms which are isotopes with non-zero nuclear spin, wherein said isotopes with non-zero nuclear spin are enriched as compared to the natural occurrence thereof. Isotopes are nuclides with the same atomic number but different mass number. The most common isotopes with non-zero nuclear spin are $^{1}H$, $^{2}D$,$^{15}N$, $^{13}C$, and $^{31}P$. Preferred isotopes are $^{13}C$ and $^{15}N$.

According to the present invention, two distinct types of isotope labels are envisaged. First, the isotope label, similar to fluorophores and spin labels, is a chemical moiety which is attached to a functional group of an amino acid of the protein kinase. Any moiety which is enriched with regard to isotopes having non-zero nuclear spin is envisaged. For the purpose of attaching such isotope labels, reactive groups as described above in relation to fluorophores may be used. Alternatively, the isotope label may be an amino acid residue present in the kinase, wherein the occurrence of nuclei with non-zero nuclear spin is elevated as compared to the natural occurrence in that specific residue. Preferably,

all atoms of a given chemical element are replaced by a corresponding isotope with non-zero nuclear spin.

Nuclear magnetic resonance (NMR) is a physical phenomenon based upon the quantum mechanical magnetic properties of the nucleus of an atom. All nuclei that contain odd numbers of protons or neutrons have an intrinsic magnetic moment and angular momentum. The most commonly measured nuclei are hydrogen ($^{1}H$) (the most receptive isotope at natural abundance) and carbon ($^{13}C$), although nuclei from isotopes of many other elements (e.g. $^{113}Cd$, $^{15}N$, $^{14}N$ $^{19}F$, $^{31}P$, $^{17}O$, $^{29}Si$, $^{10}B$, $^{11}B$, $^{23}Na$, $^{35}Cl$, $^{195}Pt$) can also be observed. NMR resonant frequencies for a particular substance are directly proportional to the strength of the applied magnetic field, in accordance with the equation for the Larmor precession frequency. NMR measures magnetic nuclei by aligning them with an applied constant magnetic field and perturbing this alignment using an alternating magnetic field, those fields being orthogonal. The resulting response to the perturbing magnetic field is the phenomenon that is exploited in NMR spectroscopy and magnetic resonance imaging, which use very powerful applied magnetic fields in order to achieve high spectral resolution, details of which are described by the chemical shift and the Zeeman Effect.

The term "amino acids" has its established meaning and refers to organic molecules that have a carboxylic and an amino functional group. They are the essential building blocks of proteins. Examples of amino acids having a free thiol group are cysteine, belonging to the 20 proteinogenic amino acids, and acetyl-cysteine being a non-standard amino acid rarely occurring in natural amino acid sequences. Proteinogenic amino acids having a free side chain amino group are lysine, histidine or arginine and amino acids being aromatic amines, such as tryptophan. Pyrrolysine, 5-hydroxylysine or *o*-aminotyrosine are non-standard amino acids having a free side chain amino group. The amino acids asparagine and glutamine instead contain an amide group and are not suitable in the present invention as they are not reactive to labeling agents and are thus excluded.

Tryptophan is an aromatic amino acid having an amino group in its indole ring. Aromatic amines are weak bases and thus unprotonated at pH 7. However, they can be modified using a reactive reagent such as an isothiocyanate or sulfonyl chloride.

Small molecules may act as mediators of binding between a protein kinase domain and a second domain. To explain further, and using the example of a PH domain as a second domain, it is known that for a number of kinases, in particular those specified in part (a) of the main embodiment, such kinases have a multi-domain structure comprising two or more domains, said domains including or being a protein kinase domain and a PH domain, wherein said PH domain, when bound to said protein kinase domain, exerts an inhibitory effect on the catalytic activity of the protein kinase domain. Certain small molecules are capable of shifting the equilibrium in favour of the bound, i.e., the inactive state of kinases comprising a protein kinase domain and a PH domain. So far, such small molecules have been discovered only to a limited extent and by chance. The present inventors for the first time provide a robust and systematic approach for the identification of such mediators of binding between a protein kinase domain and a second domain. This principle is illustrated in the examples enclosed herewith. The formation of the bound state between protein kinase domain and second domain involves the formation of contacts between the two domains, thereby defining an interface; see also above. Since said interface typically is made of regions of the two domains which are not as conserved as the ATP binding site of the protein kinase domain, a screen for small molecules which mediate binding between the two domains is advantageous in that such screen is characterized by a greater likelihood of identifying inhibitors specific for a given kinase. In fact, as evidenced by the examples enclosed herewith, an exemplary implementation of the screen according to the present invention does not identify ATP pocket binders but does identify mediators of inter-domain interaction. As is known in the art, ATP analogues or other compounds binding to the ATP binding site are likely to exhibit severe side effects even when developed into pharmaceuticals owing to their likelihood of binding to the ATP binding sites in more than one kinase.

In order to obtain a vehicle for the above described screen, the present inventors developed a polypeptide in accordance with the first aspect of the invention described herein. Said polypeptide is labeled in such a manner that a readout is obtained in case a test compound is capable of mediating the binding between the protein kinase domain and the second domain. It is noteworthy that fluorophors such as acrylan so far have only been used to detect the binding of molecules close to it, in other words when the fluorophor directly senses a change in the environment from the binding event itself. The new approach developed by the present inventors is fundamentally different in that it has surprisingly been found that the fluorophor, spin label or isotope label turned out to be sensitive to an environmental change triggered by a protein conformation change which in turn is induced by a binding event at significant distance from the fluorophor, spin label or isotope label. In fact, the distance between fluorophor, spin label or isotope label on the one hand and the binding molecule on the other hand surprisingly does not matter. This has not been reported so far. A skilled person would not have expected that fluorophor, spin labels and isotope labels may act as such "remote sensors" of a binding event.

Screening methods are the subject of the second and third aspect of this invention, respectively. The present application furthermore includes proof-of-principle compounds, i.e., compounds which have been identified using the screen in accordance with the present invention, which screen makes use of the polypeptide according to the present invention, and which compounds mediate formation of the bound and inactive state of said protein kinase domain and

said second domain.

As will be described in more detail below in relation to further embodiments of the invention, the labeled polypeptides according to the invention can be used to measure kinetics and thermodynamics of ligand binding and inhibitor binding. Moreover, screening assays using the labeled polypeptides according to the present invention can easily be adapted to high throughput screening methods.

In a preferred embodiment, (a) exactly one second domain is present; and/or (b) for each one of one, more or all of the interfaces exactly one label is present within a distance as defined above, and wherein the second domain(s) carries/carry said exactly one label.

Using the example of the PH domain being the second domain, it is accordingly preferred that the PH domain carries exactly one environmentally sensitive label within the indicated distance from said interface. The corresponding part of the protein kinase domain (1 to 15 Å from said interface or any preferred sub-range) would be free of any environmentally sensitive label. It is furthermore preferred that the polypeptide as a whole contains exactly one environmentally sensitive label, said exactly one environmentally sensitive label being placed within 1 to 15 Å (or any preferred sub-range thereof) from the interface and within said second domain.

In a preferred embodiment, said polypeptide is a kinase of the Akt family, preferably Akt1, or a fragment thereof, said fragment comprising said KD and a PH domain. This preferred embodiment closely tracks the subject-matter of the examples enclosed herewith.

In a preferred embodiment, said label is the only fluorophore, spin label or isotope label present in said polypeptide.

In a further preferred embodiment the amino acid sequence of said polypeptide is (a) the amino acid sequence of the wild type form of said polypeptide or of a fragment thereof, said fragment comprising said KD and said second domain; or (b)(i) differs from the amino acid sequence of said wild type form or fragment thereof by the presence of one amino acid residue containing a thiol group or side chain amino group at a position where the amino acid sequence of said wild type form does not contain such a residue, said residue being within a distance from said interface as defined above; and/or (ii) differs from the amino acid sequence of said wild type form or fragment thereof by the absence of one or more amino acid residues containing a thiol group or side chain amino group, provided that exactly one amino acid residue, preferably exactly one solvent-exposed amino acid residue containing a thiol group or side chain amino group is present in said polypeptide within a distance from said interface as defined above.

Depending on the amino acid residues being present in the wild type form of a given kinase or fragment thereof, said fragment comprising KD and second domain, either the wild type form or fragment as such may be amenable to labeling, or certain sequence modifications may be required in order to introduce an amino acid residue amenable to labeling. The recited presence of one amino acid residue according to option (b)(i) of this embodiment may be achieved either by substitution or insertion of said one amino acid residue. Similarly, the absence of one or more amino acid residues according to option (b)(ii) may be achieved by substitution of more or more amino acid residues or deletion of one or more amino acid residues, in either case resulting in the removal of one or more amino acid residues containing a thiol group or side chain amino group. For any of these sequence modifications, means and methods are available at the skilled person's disposal. Such methods include site-directed mutagenesis as well as other recombinant synthetic or semi-synthetic techniques. In case any of the above described non-standard amino acids is to be introduced into the kinase, an amino acid stretch containing said amino acid may be chemically synthesized and then connected to the remaining part(s) of the kinase which may have been produced recombinantly or synthetically. Alternatively, kinases expressed and designed to incorporate a specialized non-standard amino acid at the desired position for subsequent labeling may be produced recombinantly by applying an altered genetic code (see e. g. Liu and Schultz, 2010). Preferred means for achieving presence of an amino acid according to option (b)(i) are substitution, and the preferred means for achieving absence of one or more amino acid residues according to option (b)(ii) is substitution as well.

The term "delete" refers to excision of an amino acid without replacing it with another amino acid whereas the term "replace" refers to the substitution of an amino acid with another amino acid.

Preferably, amino acid replacements should be conservative. For cysteine, this means that it is preferably replaced with serine. In general, replacements of amino acids with different amino acids may be evaluated in view of whether they are conservative using the PAM250 Scoring matrix. The matrix is frequently used to score aligned peptide sequences to determine the similarity of those sequences (Pearson, 1990). More generally, amino acids that have the closest resemblance to cysteine are preferred so that an artificial cysteine mutation will have the least impact on the kinase, its structure and catalytic activity (most preferred alanine and leucine, less preferred serine, threonine and methionine (serine and threonine only to the extent they are not phosphorylation sites - in that case they would be important for regulation of the labelled kinase and should preferably not be replaced with cysteine), and yet less preferred are isoleucine, valine, and glycine).

In a further preferred embodiment, (a) the amino acid sequence of said polypeptide outside said region within 1 to 15 Å from said interface or a preferred sub-range thereof, is that of the wild type form; or (b) one, more or all amino acid residues containing a thiol group or side chain amino group and outside said region are deleted or replaced with

amino acid residues which do not contain a thiol group or side chain amino group. According to option (b), those amino acids which are potentially amenable to labeling (amino acid residues containing a thiol group or side chain amino group) are deleted or replaced with amino acid residues which are not amenable to labeling. This is a preferred measure which ensures that exactly one or exactly two sites, respectively, said site(s) being located in said region, is/are labeled, said site(s) being the only labeled site(s) in the polypeptide as a whole.

In a preferred embodiment, said amino acid residues containing a thiol group or a side chain amino group and outside said region according to (b) are solvent-exposed. As is known in the art, solvent-exposed amino acids, to the extent they contain suitable functional groups (thiol or side chain amino groups), are more likely to react with a reactive agent. Therefore, it is preferred that solvent-exposed amino acids, more specifically only solvent-exposed amino acids containing a thiol group or a side chain amino group and outside said region are deleted or replaced as defined according to (b) of the preceding embodiment.

The term "solvent-exposed" refers to the position of an amino acid in the context of the three dimensional structure of the protein of which it is a part. Amino acids buried within the protein body are completely surrounded by other amino acids and thus do not have contact with the solvent. In contrast, solvent-exposed amino acids are partially or fully exposed to the surrounding solvent and are thus accessible to chemicals potentially able to modify them. This applies e.g. to thiol- or amino-reactive labels used in the present invention which can react with solvent-exposed amino acids having a free thiol- or amino-group.

The determination of solvent-exposed amino acid residues can be performed by a skilled person without further ado. For example, the wild type form of the kinase can be contacted with agents reacting with thiol groups and or side chain amino groups. Subsequently, the labeled positions may be determined, thereby determining the solvent-exposed amino acid residues containing a thiol group or side chain amino group. Alternatively or in addition, computational means may be used. Such computational means make us of the three-dimensional structure of the protein kinase, said three-dimensional structure being either experimentally determined (such as by X-ray crystallography or NMR), or predicted by methods such as homology modeling. Provided with the coordinates of the three-dimensional structure, the skilled person may use established bioinformatic tools which determine solvent exposure of each individual atom as well as of residues in their entirety.

In a further preferred embodiment, said polypeptide differs from the amino acid sequence of said wild type form or fragment thereof by the presence of exactly one amino acid residue containing a thiol group or side chain amino group at a position where the amino acid sequence of the wild type does not contain such a residue. This embodiment relates to a labeled polypeptide according to the present invention which has the amino acid sequence of the wild type form outside said region, and exactly one amino acid residue amenable to labeling, i.e., an amino acid residue containing a thiol group or a side chain amino group within the said region, wherein said amino acid residue amenable to labeling is not present in said region in the wild type form of said kinase. The same considerations apply *mutatis mutandis* to those embodiments where exactly two labels are present.

In a more preferred embodiment, said exactly one amino acid residue is a cysteine replacing an amino acid residue of said wild type form.

In a second aspect, the present invention provides a method of screening for ligands of a second polypeptide, said second polypeptide being (i) identical to the polypeptide according to the first aspect; (ii) the wild type form of said polypeptide according to the first aspect; or (iii) a fragment of the polypeptide of (i) or (ii), said fragment comprising the KD and the second domain as comprised in the polypeptide of (i); said method comprising: (a) contacting the polypeptide according to the first aspect with a candidate compound; and (b) recording the fluorescence emission signal, the EPR signal or the NMR signal of said label defined above, wherein a difference in signal as compared to the signal in absence of said candidate compound is indicative of said candidate compound being a ligand of said second polypeptide.

The term "second polypeptide" is used herein to distinguish a naturally occurring, preferably disease-relevant molecule (such molecule being a preferred embodiment of such second polypeptide) from the labeled and potentially sequence-modified counterpart thereof which counterpart is a polypeptide according to the first aspect of the present invention. In other words, using a polypeptide according to the first aspect as a vehicle in the screen may be used to identify a ligand of the naturally occurring counterpart thereof.

The term "ligand" has the meaning as established in the art. It refers to any compound which directly interacts or binds to a kinase. Preferably, the equilibrium binding constant is in the millimolar, nanomolar, picomolar or below range. It is understood that in the course of the screening typically one or more of the tested compounds is discarded because it does not exhibit any measurable binding.

Said contacting according to (a) occurs under conditions which allow binding of a ligand known to be capable of binding to said kinase. The skilled person, being acquainted with the handling of kinases as well as the design of assays using kinases as a target, can establish such suitable conditions without further ado. Such conditions include buffered solutions.

Depending on whether the label present in the kinase according to the invention is a fluorophore, spin label or an isotope label, step (b) of the method according to the invention provides for recording the fluorescence emission

signal, recording the EPR signal, or recording the NMR signal, respectively.

It is understood that the term "signal" embraces a measurement at a single wavelength as well as a spectral scan. Examples of a fluorescence spectrum, and how this spectrum is subjected to change upon addition of a ligand, are shown in of Figure 2 as enclosed herewith. In case for a given labeled kinase it is not yet known where in the spectrum the maximum change in signal occurs upon ligand binding, it will in general be advisable to perform a spectral scan in order to determine the wavelength of maximum change upon ligand binding. If this is already known, performing the measurement at a single wavelength, preferably at the wave length of maximum change, is sufficient. A "difference in signal" refers to a change of the entire fluorescence emission spectrum, EPR spectrum or NMR spectrum, a shift of the maximum emission wavelength, and/or a change of emission at the given wavelength.

Adding a further fluorophore to the solution in case the label is a fluorophore, the further fluorophore being neither attached to the protein nor having to be environment-sensitive, but amenable to excitation under the same conditions as said label but having a different emission spectrum, can serve as an intensity reference and make the assay readout more reliable (e.g. adding Texas Red for an eosin label).

In a third aspect, the present invention provides a method of screening for inhibitors of kinase activity of a second polypeptide said second polypeptide being (i) identical to the polypeptide according to the first aspect; (ii) the wild type form of said polypeptide according to the first aspect; or (iii) a fragment of the polypeptide of (i) or (ii), said fragment comprising the KD and the second domain as comprised in the polypeptide of (i); said method comprising the method according to the second aspect and (c) determining the kinase activity of the KD comprised in the second polypeptide in the presence of said candidate compound, wherein a decrease in activity as compared to the absence of said candidate compound is indicative of said candidate compound being an inhibitor of said kinase activity.

This aspect of the present invention provides for screening for specific types of ligands which are inhibitors of kinase activity. Accordingly, the method includes the further step of monitoring the activity of the kinase. Generally speaking, the activity of the kinase may be the biochemical activity, said biochemical activity embracing phosphorylating activity with regard to the target of the kinase. Alternatively or in addition, the activity at a cellular level may be determined. This includes readouts not at the level of the kinase itself, but instead, for example, of an entire pathway comprising the kinase at issue. If the kinase is disease-relevant, disease models may be used for monitoring activity. These and further kinase activity assays are within the skills of the skilled person.

Kinase inhibitors are substances capable of inhibiting the activity of kinases. The present invention provides means and methods for identifying kinase inhibitors which, instead of binding to conserved regions of the protein kinase domain such as the ATP binding site, mediate inter domain interactions and thereby achieve their inhibitory effect.

A candidate inhibitor may belong to different classes of compounds such as small organic or inorganic molecules, proteins or peptides, nucleic acids such as DNA or RNA. Such compounds can be present in molecule libraries or designed from scratch.

Small molecules according to the present invention comprise molecules with a molecular weight of up to 2000 Da, preferably up to 1500 Da, more preferably up to 1000 Da and most preferably up to 500 Da.

Preferably, the method of screening according to the invention is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact with test compounds, in this case putative inhibitors, with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits inhibitory activity, said mixture of test inhibitors may be de-convoluted to identify the one or more test inhibitors in said mixture giving rise to said activity.

Alternatively, only one test inhibitor may be added to a well, wherein each test inhibitor is applied in different concentrations. For example, the test inhibitor may be tested in two, three or four wells in different concentrations. In this initial screening, the concentrations may cover a broad range, e.g. from 10 nM to 10 μM. The initial screening serves to find hits, i.e. test inhibitors exerting inhibiting activity at at least one concentration, preferably two, more preferably all concentrations applied, wherein the hit is more promising if the concentration at which an inhibitory activity can be detected is in the lower range. This alternative serves as one preferred embodiment in accordance with the invention.

In a further aspect, the present invention provides a compound of the following formula (I)

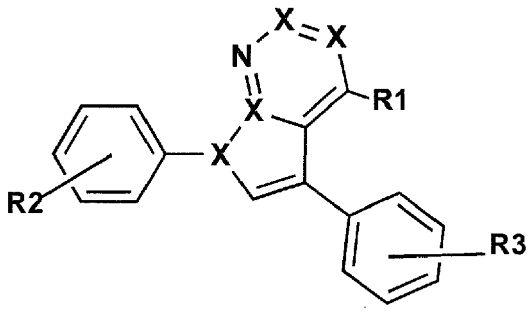

wherein X is C, N, O or S, any free valence of C, N, O or S being filled with hydrogen; $R^1$ is arylalkyl-Y, heteroarylalkyl-Y, aryl-Y, heteroaryl-Y, arylalkenyl-Y or heteroarylalkenyl-Y; aryl and heteroaryl being 5- or 6-membered rings, heteroatoms in said heteroaryl being selected from S, N and O, the number of said heteroatoms being 1, 2 or 3, alkyl being $C_1$ to $C_4$ alkyl, alkenyl being $C_1$ to $C_4$ alkenyl, Y being selected from $CH_2$, NH, SH and O; $R^2$ and $R^3$ are independently either absent or selected from halogen and $C_1$ to $C_4$ alkyl wherein the number of occurrences of $R^2$ and $R^3$ is independently selected from 1, 2 and 3.

A preferred heteroatom X is N. Preferred $R^1$ groups are heteroarylmethyl-Y, and arylmethyl-Y. Preferred arylmethyl-Y groups include benzyl. Preferred heteroaryl groups include thiophen-2-yl methyl, pyridine-2-yl methyl and pyridine-3-yl methyl. A preferred moiety Y is NH.

Preferably, said compound has the following formula (II)

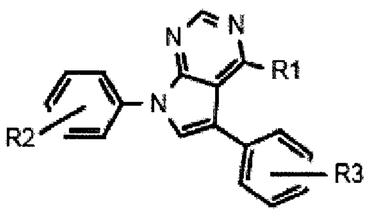

$R^1$, $R^2$ and $R^3$ are as defined above.

In a further preferred embodiment, the alkyl moiety in said arylalkyl or heteroarylalkyl is $CH_2$ or $(CH_2)_2$. Particularly preferred is $CH_2$.

In a more preferred embodiment, said compound has the following formula (III)

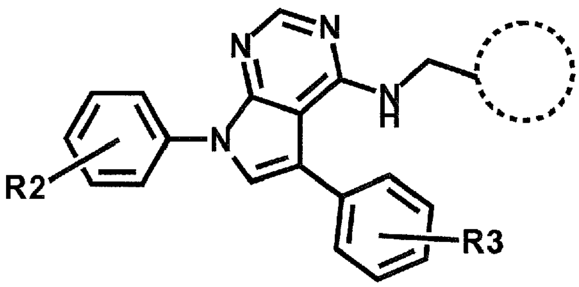

$R^2$ and $R^3$ are as defined above. The dotted circle designates said aryl or heteroaryl moiety as defined further above.

It is furthermore preferred that alkyl in said arylalkyl or heteroarylalkyl is $CH_2$.

In a further preferred embodiment, there is no occurrence of $R^3$.

A particular preferred embodiment is that of formula (IV)

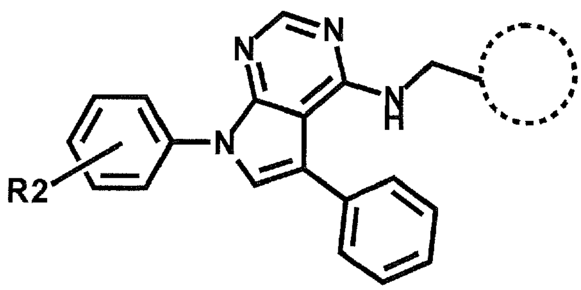

The dotted circle has the meaning as defined above. Preference is given to one or two occurrences of $R^2$, in particular to one occurrence of $R^2$. In case of one occurrence of $R^2$, meta- and para-substitution is preferred, para-substitution being especially preferred. In case of two occurrences of $R^2$, it is preferred that one is located in the para-position and the second in meta-position.

Particularly preferred compounds according to the present invention are given below.

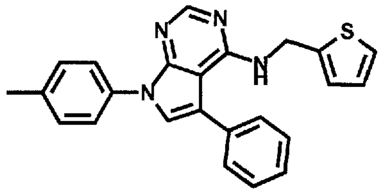

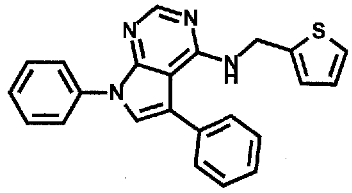

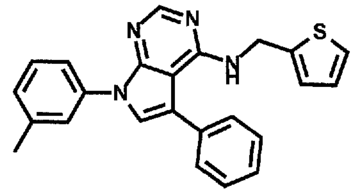

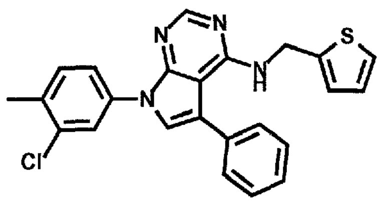

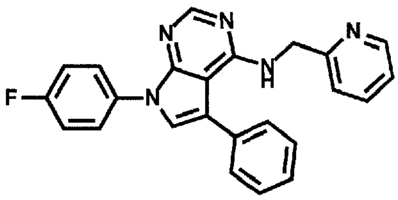

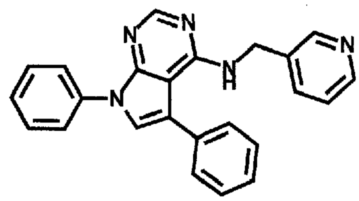

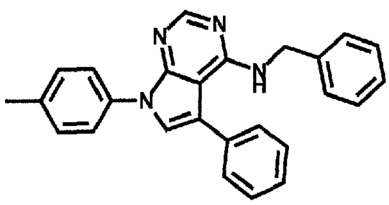

In a further aspect, the present invention provides a pharmaceutical composition comprising a compound of formula (I) or a compound in accordance with preferred embodiments thereof, said preferred embodiments being defined herein above.

The pharmaceutical composition may comprise or consist of one or more compounds in accordance with the present invention. In case it comprises one or more compounds according to the present invention, further constituents may be present. These further constituents may be pharmaceutically active compounds. Having said that, it is preferred that one or more compounds in accordance with the present invention are the only pharmaceutically active compound(s) comprised in said pharmaceutical composition.

To the extent said pharmaceutical composition comprises one or more compounds according to the present invention, it is furthermore envisaged that carriers, excipients and/or fillers as known in the pharmaceutical art are present.

More specifically, the pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The effective amount of the pharmaceutical composition for purposes herein is thus determined by such considerations.

The skilled person knows that the effective amount of pharmaceutical composition administered to an individual will, inter alia, depend on the nature of the compound. For example, the total pharmaceutically effective amount of

pharmaceutical composition administered parenterally per dose may be in the range of about 1 μg compound/kg/day to 10 mg protein /kg/day of patient body weight, although, as noted above, this will be subject to therapeutic discretion. More preferably, this dose is at least 0.01 mg compound/kg/day, and most preferably for humans between about 0.01 and 1 mg compound/kg/day. If given continuously, the pharmaceutical composition is typically administered at a dose rate of about 1 μg/kg/hour to about 50 μg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

Pharmaceutical compositions of the invention may be administered orally, rectally, parenterally, intracisternally, intravaginally, intraperitoneally, topically (as by powders, ointments, drops or transdermal patch), bucally, or as an oral or nasal spray.

Pharmaceutical compositions of the invention preferably comprise a pharmaceutically acceptable carrier. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semi-permeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Biopolymers 22:547-556 (1983)), poly (2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The solution for infusion or injection is prepared by reconstituting the lyophilized compound(s) using bacteriostatic Water-for-Injection.

In a further aspect, the present invention provides a compound as defined herein above for use in a method of treating cancer.

Preferred cancers according to the present invention includes Locally Advanced Tumors; Metastatic Solid Tumors; Cancer; Neoplasms, Locally Advanced, Metastatic Solid Tumors Estrogen Receptor-positive Breast Cancer;

Recurrent Breast Cancer; Stage IV Breast Cancer, Solid Tumors, Non Small Cell Lung Cancer, Ovarian Cancer; Fallopian Tube Cancer; Primary Peritoneal Cancer, Acinar Cell Adenocarcinoma of the Pancreas; Duct Cell Adenocarcinoma of the Pancreas; Recurrent Pancreatic Cancer; Stage III Pancreatic Cancer; Stage IV Pancreatic Cancer, B-cell Chronic Lymphocytic Leukemia; Recurrent Small Lymphocytic Lymphoma; Refractory Chronic Lymphocytic Leukemia; Stage I Chronic Lymphocytic Leukemia; Stage II Chronic Lymphocytic Leukemia; Stage III Chronic Lymphocytic Leukemia; Stage IV Chronic Lymphocytic Leukemia Estrogen Receptor-negative Breast Cancer; Estrogen Receptor-positive Breast Cancer; HER2-positive Breast Cancer; Progesterone Receptor-negative Breast Cancer; Progesterone Receptor-positive Breast Cancer; Recurrent Breast Cancer; Stage IV Breast Cancer, HER2-positive Breast Cancer; Male Breast Cancer; Recurrent Breast Cancer; Stage IIIB Breast Cancer; Stage IIIC Breast Cancer; Stage IV Breast Cancer; Unspecified Adult Solid Tumor, Protocol Specific Adenocarcinoma of the Gastroesophageal Junction; Adenocarcinoma of the Stomach; Recurrent Esophageal Cancer; Recurrent Gastric Cancer, Advanced Solid Tumors; Breast Cancer, Carcinoma Breast Stage IV, PANCREAS; Neuroendocrine, Recurrent Prostate Cancer; Recurrent Renal Cell Cancer; Stage III Prostate Cancer; Stage III Renal Cell Cancer; Stage IV Prostate Cancer; Stage IV Renal Cell Cancer; Unspecified Adult Solid Tumor, Protocol Specific, Adenocarcinoma of the Gastroesophageal Junction; HER2-positive Breast Cancer; Male Breast Cancer; Recurrent Breast Cancer; Recurrent Esophageal Cancer; Recurrent Gastric Cancer; Stage IIIC Breast Cancer; Stage IIIC Esophageal Cancer; Stage IIIC Gastric Cancer; Stage IV Breast Cancer; Stage IV Esophageal Cancer; Stage IV Gastric Cancer, Recurrent Prostate Cancer; Stage I Prostate Cancer; Stage IIA Prostate Cancer; Stage IIB Prostate Cancer; Stage III Prostate Cancer, Malignant Glioma, Recurrent Adenoid Cystic Carcinoma of the Oral Cavity; Recurrent Salivary Gland Cancer; Salivary Gland Adenoid Cystic Carcinoma; Stage IVA Adenoid Cystic Carcinoma of the Oral Cavity; Stage IVA Salivary Gland Cancer; Stage IVB Adenoid Cystic Carcinoma of the Oral Cavity; Stage IVB Salivary Gland Cancer; Stage IVC Adenoid Cystic Carcinoma of the Oral Cavity, Colon Cancer; Rectal Cancer, Adenocarcinoma of the Lung; Adenosquamous Cell Lung Cancer; Bronchoalveolar Cell Lung Cancer; Large Cell Lung Cancer; Recurrent Non-small Cell Lung Cancer; Squamous Cell Lung Cancer, Diffuse Large B Cell Lymphoma, Recurrent Breast Cancer; Stage IV Breast Cancer; Unspecified Adult Solid Tumor, Protocol Specific Estrogen Receptor-positive Breast Cancer; HER2-negative Breast Cancer; Recurrent Breast Cancer; Stage II Breast Cancer; Stage IIIA Breast Cancer; Stage IIIB Breast Cancer; Stage IIIC Breast Cancer, Colorectal Neoplasms, Acute Undifferentiated Leukemia; Angioimmunoblastic T-cell Lymphoma; Childhood Burkitt Lymphoma; Childhood Chronic Myelogenous Leukemia; Childhood Diffuse Large Cell Lymphoma; Childhood Grade III Lymphomatoid Granulomatosis; Childhood Immunoblastic Large Cell Lymphoma; Childhood Nasal Type Extranodal NK/T-cell Lymphoma; Cutaneous B-cell Non-Hodgkin Lymphoma; Extranodal Marginal Zone B-cell Lymphoma of Mucosa-associated Lymphoid Tissue; Intraocular Lymphoma; Juvenile Myelomonocytic Leukemia; Mast Cell Leukemia; Nodal Marginal Zone B-cell Lymphoma; Post-transplant Lymphoproliferative Disorder; Primary Central Nervous System Hodgkin Lymphoma; Primary Central Nervous System Non-Hodgkin Lymphoma; Progressive Hairy Cell Leukemia, Initial Treatment; Prolymphocytic Leukemia; Recurrent Childhood Acute Lymphoblastic Leukemia; Recurrent Childhood Acute Myeloid Leukemia; Recurrent Childhood Anaplastic Large Cell Lymphoma; Recurrent Childhood Grade III Lymphomatoid Granulomatosis; Recurrent Childhood Large Cell Lymphoma; Recurrent Childhood Lymphoblastic Lymphoma; Recurrent Childhood Small Noncleaved Cell Lymphoma; Recurrent Cutaneous T-cell Non-Hodgkin Lymphoma; Recurrent Grade 1 Follicular Lymphoma; Recurrent Grade 2 Follicular Lymphoma; Recurrent Grade 3 Follicular Lymphoma; Recurrent Mantle Cell Lymphoma; Recurrent Marginal Zone Lymphoma; Recurrent Mycosis Fungoides/Sezary Syndrome; Recurrent Small Lymphocytic Lymphoma; Recurrent/Refractory Childhood Hodgkin Lymphoma; Refractory Chronic Lymphocytic Leukemia; Refractory Hairy Cell Leukemia; Relapsing Chronic Myelogenous Leukemia; Secondary Acute Myeloid Leukemia; Small Intestine Lymphoma; Splenic Marginal Zone Lymphoma; T-cell Large Granular Lymphocyte Leukemia; Unspecified Childhood Solid Tumor, Protocol Specific; Waldenström Macroglobulinemia, Neoplasms, Malignant Adult Acute Megakaryoblastic Leukemia (M7); Adult Acute Minimally Differentiated Myeloid Leukemia (M0); Adult Acute Monoblastic Leukemia (M5a); Adult Acute Monocytic Leukemia (M5b); Adult Acute Myeloblastic Leukemia With Maturation (M2); Adult Acute Myeloblastic Leukemia Without Maturation (M1); Adult Acute Myeloid Leukemia With 11q23 (MLL) Abnormalities; Adult Acute Myeloid Leukemia With Del(5q); Adult Acute Myeloid Leukemia With Inv(16)(p13;q22); Adult Acute Myeloid Leukemia With t(16;16)(p13;q22); Adult Acute Myeloid Leukemia With t(8;21)(q22;q22); Adult Acute Myelomonocytic Leukemia (M4); Adult Erythroleukemia (M6a); Adult Pure Erythroid Leukemia (M6b); Recurrent Adult Acute Myeloid Leukemia, Adenocarcinoma of the Pancreas; Recurrent Pancreatic Cancer; Stage IV Pancreatic Cancer, Recurrent Melanoma; Stage IIIA Melanoma; Stage IIIB Melanoma; Stage IIIC Melanoma; Stage IV Melanoma, Recurrent Squamous Cell Carcinoma of the Nasopharynx; Stage IV Squamous Cell Carcinoma of the Nasopharynx, Adult Grade III Lymphomatoid Granulomatosis; Adult Nasal Type Extranodal NK/T-cell Lymphoma; Anaplastic Large Cell Lymphoma; Angioimmunoblastic T-cell Lymphoma; Cutaneous B-cell Non-Hodgkin Lymphoma; Extranodal Marginal Zone B-cell Lymphoma of Mucosa-associated Lymphoid Tissue; Hepatosplenic T-cell Lymphoma; Intraocular Lymphoma; Nodal Marginal Zone B-cell Lymphoma; Noncutaneous Extranodal Lymphoma; Peripheral T-cell Lymphoma; Recurrent Adult Diffuse Large Cell Lymphoma; Recurrent Adult Diffuse Mixed Cell Lymphoma; Recurrent Adult Diffuse Small Cleaved Cell Lymphoma; Recurrent Adult Grade III Lymphomatoid Granulomatosis; Recurrent Adult Hodgkin Lymphoma; Recurrent Adult Immunoblastic Large Cell Lymphoma; Recurrent

Adult T-cell Leukemia/Lymphoma; Recurrent Grade 1 Follicular Lymphoma; Recurrent Grade 2 Follicular Lymphoma; Recurrent Grade 3 Follicular Lymphoma; Recurrent Mantle Cell Lymphoma; Recurrent Marginal Zone Lymphoma; Recurrent Mycosis Fungoides/Sezary Syndrome; Recurrent Small Lymphocytic Lymphoma; Refractory Hairy Cell Leukemia; Small Intestine Lymphoma; Splenic Marginal Zone Lymphoma; T-cell Large Granular Lymphocyte Leukemia; Testicular Lymphoma; Waldenström Macroglobulinemia, Endometrial Adenocarcinoma; Endometrial Adenosquamous Cell Carcinoma; Endometrial Clear Cell Carcinoma; Endometrial Papillary Serous Carcinoma; Recurrent Endometrial Carcinoma, Adult Primary Hepatocellular Carcinoma; Advanced Adult Primary Liver Cancer; Localized Unresectable Adult Primary Liver Cancer; Recurrent Adult Primary Liver Cancer, Carcinoma Breast Stage IV, Adult Grade III Lymphomatoid Granulomatosis; Extranodal Marginal Zone B-cell Lymphoma of Mucosa-associated Lymphoid Tissue; Intraocular Lymphoma; Nodal Marginal Zone B-cell Lymphoma; Recurrent Adult Diffuse Large Cell Lymphoma; Secondary Central Nervous System Non-Hodgkin Lymphoma; Small Intestine Lymphoma; Splenic Marginal Zone Lymphoma; Testicular Lymphoma; Waldenström Macroglobulinemia, Mucinous Adenocarcinoma of the Colon; Mucinous Adenocarcinoma of the Rectum; Recurrent Colon Cancer; Recurrent Rectal Cancer; Signet Ring Adenocarcinoma of the Colon; Signet Ring Adenocarcinoma of the Rectum; Stage IIIA Colon Cancer; Stage IIIA Rectal Cancer; Stage IIIB Colon Cancer; Stage IIIB Rectal Cancer; Stage IIIC Colon Cancer; Stage IIIC Rectal Cancer; Stage IVA Colon Cancer; Stage IVA Rectal Cancer; Stage IVB Colon Cancer; Stage IVB Rectal Cancer, Advanced Cancer, Male Breast Cancer; Recurrent Breast Cancer; Stage IIIB Breast Cancer; Stage IIIC Breast Cancer; Stage IV Breast Cancer, Advanced Adult Primary Liver Cancer; Localized Unresectable Adult Primary Liver Cancer; Metastatic Extrahepatic Bile Duct Cancer; Metastatic Gallbladder Cancer; Recurrent Adult Primary Liver Cancer; Recurrent Gallbladder Cancer; Unresectable Extrahepatic Bile Duct Cancer; Unresectable Gallbladder Cancer, Endometrial Cancer, Clear Cell Renal Cell Carcinoma; Recurrent Renal Cell Cancer; Stage III Renal Cell Cancer; Stage IV Renal Cell Cancer; Type 1 Papillary Renal Cell Carcinoma; Type 2 Papillary Renal Cell Carcinoma, Advanced Solid Tumors; Tumors; Cancer, Locally Advanced or Metastatic Solid Tumors, Estrogen Receptor-negative Breast Cancer; Estrogen Receptor-positive Breast Cancer; HER2-negative Breast Cancer; HER2-positive Breast Cancer; Progesterone Receptor-negative Breast Cancer; Progesterone Receptor-positive Breast Cancer; Stage IB Breast Cancer; Stage II Breast Cancer; Stage IIIA Breast Cancer; Stage IIIB Breast Cancer; Stage IIIC Breast Cancer; Triple-negative Breast Cancer, Recurrent Nasopharyngeal Carcinoma, Lung Cancer, Carcinoma, Non-Small-Cell Lung; Carcinoma, Small Cell Lung; Carcinoma, Thymic Breast Neoplasms; Breast Cancer; Breast Tumors, Breast Cancer

In a further aspect, the present invention provides a method of quantifying ligand or inhibitor binding to a polypeptide comprising a KD and a second domain, said second domain being as defined above, said method comprising (a)(i) contacting a polypeptide according to the first aspect with different concentrations of a ligand or inhibitor; and/or (ii) contacting a complex, the complex comprising or consisting of the polypeptide according to the first aspect and a ligand or inhibitor, with different concentrations of a second polypeptide as defined above; (b) recording the signal emitted by the label of said polypeptide according to the first aspect at a given time point after said contacting and for each concentration of ligand or inhibitor according to (a)(i) and/or for each concentration of said second polypeptide according to (a)(ii); and (c) determining the dissociation and/or association constant of said complex from the concentration dependence of said signal recorded according to (b).

This aspect of the present invention refers to so-called endpoint measurements. This is reflected by the feature "at a given time point after said contacting" as required by step (b) of the method. This method allows to determine the equilibrium dissociation constant $K_d$ of the complex formed by the kinase and the ligand. The association constant $K_a$ is related to the dissociation constant according to $K_a=1/K_d$.

The term "quantifying ligand or inhibitor binding to a kinase" refers to the determination of kinetic and/or thermodynamic parameters governing the interaction between the kinase and the ligand or inhibitor.

As previously discussed in relation to other embodiments, said contacting occurs under conditions which allow binding of a known ligand to a given kinase.

Suitable time points after said contacting can be determined by the skilled person without further ado.

The end point signal, when plotted as a function of the logarithm of the concentration according to (a)(i) or (a)(ii), usually has sigmoidal shape. The concentration corresponding to the inflection point of the sigmoidal curve corresponds to the value of $K_d$. Measuring the same samples over time and making several such curves will reveal the time needed. As the compounds bind slowly, the signal changes and the corresponding curve will shift leftward with time until it reaches its final value - this is the true $K_d$ curve for the compound at equilibrium.

In a preferred embodiment, said label is a fluorophore and said signal is the ratio of emission intensities at two different wavelengths, the two wavelengths being local maxima of the emission spectrum. This ratio provides a sensitive measure of changes in the spectrum.

This preferred embodiment is a specific implementation of the general method of quantifying ligand or inhibitor binding according to the present invention, wherein said preferred embodiment relates to those labeled kinases wherein the label is a fluorophore.

Alternatively or in addition, further parameters may be determined. These parameters include the ratio of the

normalized intensity change to the average intensity change ($\Delta I_{std}$), the maximum standard intensity change ($\Delta R_{max}$) as well as the Z-factor. These quantities are defined and discussed below. A labeled kinase according to the invention, particularly a fluorescently labeled kinase according to the invention is considered particularly suitable for the screening of the kinase inhibitors if ($\Delta I_{std}$) is > 0.25, and/or ($\Delta R_{max}$) is > 0.75 and the Z-factor is > 0.5.

$\Delta I_{std}$ is the ratio of normalized intensity change to average intensity of the fluorescence emission. According to de Lorimier et al. (2002), $\Delta I_{std}$ is one of the most important criteria for characterizing a fluorescent protein conjugate as suitable for sensitive fluorescence spectroscopy. Ideally, the $\Delta I_{std}$ should have a value > 0.25 and is calculated by:

$$\Delta I_{std} = \left| \frac{2(I_1(\lambda_{std}) - I_2(\lambda_{std}))}{I_1(\lambda_{std}) + I_2(\lambda_{std})} \right|$$

where λstd = (λmax, unbound + λmax, saturated)/2 and I1, I2 are the fluorescence intensities at λstd of each spectrum respectively.

$\Delta R_{max}$ is the maximum standard intensity change of the fluorescence emission between saturated and unsaturated kinase. According to (de Lorimier et al., 2002), $\Delta R_{max}$ is another important criteria for characterizing a fluorescent protein conjugate as suitable for sensitive fluorescence spectroscopy. Ideally, the $\Delta R_{max}$ should have a value > 1.25 and is calculated by:

$$\Delta R = \left| \frac{{}^{0}A_1}{{}^{0}A_2} - \frac{{}^{\infty}A_1}{{}^{\infty}A_2} \right|$$

where $^{o}$A1, $^{o}$A2 are the areas in the absence of ligand, and $^{\infty}$A1, $^{\infty}$A2 are the areas in the presence of saturating ligand. A computer program can be used to enumerate ΔR for all possible pairs of wavelength bands in the two spectra, to identify the optimal sensing condition, defined as the maximum value of ΔR.

The Z-factor is a statistical measure of the quality or power of a high-throughput screening (HTS) assay. In an HTS campaign, large numbers of single measurements of unknown samples are compared to well established positive and negative control samples to determine which, if any, of the single measurements are significantly different from the negative control. Prior to starting a large screening campaign, much work is done to assess the quality of an assay on a smaller scale, and predict if the assay would be useful in a high-throughput setting. The Z-factor predicts if useful data could be expected if the assay were scaled up to millions of samples. The Z- factor is calculated by:

$$Zfactor = 1 - \frac{3 \times (\sigma_p + \sigma_n)}{|\mu_p - \mu_n|}$$

wherein both the mean (μ) and standard deviation (σ) of both the positive (p) and negative (n) controls ($\mu_\rho$, $\sigma_\rho$, $\mu_\eta$, $\sigma_\eta$, respectively) are taken into account.

The measurement of $\Delta I_{std}$ and $\Delta R_{max}$ as well as the determination of the Z-factor may prove useful in determining whether the label chosen is particularly suitable in the screening for inhibitors. De Lorimier discusses that the measured kinetics and $K_d$ obtained with a fluorescent tagged protein will depend on the protein, the ligand, the chosen labeling site and the fluorophore used. Therefore, the same inhibitor binding to the same labeled kinase could give different $K_d$ values depending on the label used and its position in the protein. In addition to comparing measured $K_d$ values with known values reported in literature, the determination of the above values might indicate which label should be preferred.

In a further aspect, the present invention provides a method of quantifying ligand or inhibitor binding to a kinase, said method comprising (a)(i) contacting a polypeptide according to the first aspect with at least one concentration of a ligand or inhibitor; and/or (ii) contacting a complex, the complex comprising or consisting of the polypeptide according to the first aspect and a ligand or inhibitor, with at least one concentration of a second single polypeptide as defined above; (b) recording the signal emitted by the label defined in the first aspect as a function of time upon said contacting according to (a); and (c)(i) determining the rate constant governing the time dependence of said signal according to (b) for (each of) the concentration(s) according to (a)(i) and determining $k_{on}$ therefrom; and/or (ii) determining the rate constant governing the time dependence of said signal according to (b) for (each of) the concentration(s) according to

(a)(ii) and determining $k_{off}$ therefrom.

This aspect of the invention relates to real time measurements as indicated by the feature "as a function of time" required by step (b) of the method. The thereby obtained time dependencies allow determination of the kinetic constants $k_{on}$ and $k_{off}$ by established means which is reflected in step (c) of the method according to this aspect of the invention.

In a preferred embodiment, this method of quantifying ligand or inhibitor binding further comprises calculating the dissociation constant and/or association constant from $k_{on}$ and $k_{off}$ as determined in step (c). As is well-known in the art, the association constant $K_a$ is related to the kinetic constants $k_{on}$ and $k_{off}$ as follows: $K_a = k_{on} / k_{off}$.

In chemical kinetics, a rate constant *k* quantifies the speed of a chemical reaction. For a chemical reaction where substance A and B are reacting to produce C, the reaction rate has the form:

$$\frac{d[C]}{dt} = k(T)[A]^m[B]^n$$

wherein k(T) is the reaction rate constant that depends on temperature, and [A] and [B] are the concentrations of substances A and B, respectively, in moles per volume of solution assuming the reaction is taking place throughout the volume of the solution. The exponents *m* and *n* are the orders and depend on the reaction mechanism. They can be determined experimentally.

A single-step reaction can also be described as:

$$\frac{d[C]}{dt} = Ae^{\frac{-E_a}{RT}}[A]^m[B]^n$$

$E_a$ is the activation energy and R is the Gas constant. Since at temperature *T* the molecules have energies according to a Boltzmann distribution, one can expect the proportion of collisions with energy greater than $E_a$ to vary with $e^{-E_a/RT}$. *A* is the pre-exponential factor or frequency factor.

$k_{on}$ and $k_{off}$ are constants that describe non-covalent equilibrium binding. When a ligand interacts with a receptor, or when a substrate interacts with an enzyme, the binding follows the law of mass action.

$$R + L \underset{k_{off}}{\overset{k_{on}}{\rightleftharpoons}} RL$$

In this equation R is the concentration of free receptor, L is the concentration of free ligand, and RL is the concentration of receptor-ligand complex. In the case of enzyme kinetics, R is the enzyme, or in this case a protein kinase, and L is the substrate, or in this case a candidate or known inhibitor. The association rate constant $k_{on}$ is expressed in units of $M^{-1}sec^{-1}$. The rate of RL formation equals [R] x [L] x $k_{on}$. The dissociation rate constant $k_{off}$ is expressed in units of $sec^{-1}$. The rate of RL dissociation equals [RL] x $k_{off}$. At equilibrium, the backward (dissociation) reaction equals the forward (association) reaction. Binding studies measure specific binding, which is a measure of [RL]. Enzyme kinetic assays assess enzyme velocity, which is proportional to [RL], the concentration of enzyme-substrate complexes.

$$RL = R \cdot L \cdot \frac{k_{on}}{k_{off}}$$

The equilibrium dissociation constant, $K_d$ is expressed in molar units and defined to equal $k_{off}/k_{on}$ to arrive at

$$RL = R \cdot L \cdot \frac{k_{on}}{k_{off}} = \frac{R \cdot L}{K_d}$$

The dissociation constant ($K_d$) corresponds to the concentration of ligand (L) at which the binding site on a particular protein is half occupied, i.e. the concentration of ligand, at which the concentration of protein with ligand bound (RL), equals the concentration of protein with no ligand bound (R). The smaller the dissociation constant, the more tightly bound the ligand is, or the higher the affinity between ligand and protein.

Accordingly, the association constant $K_a$, also called inverse $K_d$, is defined as $1/k_d$. The dissociation constant for a particular ligand-protein interaction can change significantly with solution conditions (e.g. temperature, pH and salt concentration).

In a further aspect, the present invention provides a method of generating a polypeptide suitable for the screening of kinase inhibitors, said method comprising (a) deleting one or more amino acid residues having a free thiol group or free side chain amino group, if any, present in a polypeptide comprising a KD and a second domain, said second domain being defined above, the labeling of said amino acid residues not being desirable, or replacing them with amino acid residues which do not contain a free thiol or side chain amino group, wherein preferably said amino acid residues the labeling of which is not desirable are solvent-exposed; (b) replacing in said polypeptide according to (a) an amino acid residue within a distance from the interface as defined above with an amino acid residue containing a free thiol or side chain amino group if no amino acid residue containing a free thiol or side chain amino group is or would be present within said distance after step (a), wherein steps (a) and (b) can be effected in any order; and (c) labeling the polypeptide obtained from steps (a) and (b) with a thiol- or amino-reactive fluorophore, thiol- or amino-reactive spin label, and/or thiol- or amino-reactive isotope label, thereby obtaining said polypeptide suitable for the screening of kinase inhibitors.

Steps (a) and (b) of this method serve to ensure that labeling according to step (c) only occurs at the position where labeling is desired.

Labeling according to step (c) makes use of thiol or amino reactive forms of the fluorophore, spin label or isotope label according to the invention.

The term "thiol- or amino-reactive" denotes the property of a compound, e.g. a fluorophore, spin label or isotope label to specifically react with free thiol- or amino groups. This is due to a functional group present in said compound which directs a specific reaction with a thiol or amino group. These functional groups may be coupled to molecules such as fluorophores, spin labels or isotope labels in order to provide labels specific for free thiol- or amino-groups. Examples for thiol-specific compounds are e.g. haloalkyl compounds such as iodoacetamide, maleimides, Hg-Link™ phenylmercury compounds or TS-link™ reagents (both Invitrogen). Haloalkyl compounds react with thiol or amino groups depending on the pH.

The process of labeling involves incubation of the kinase, optionally modified in accordance with steps (a) and (b) of the method of generating a kinase, with a thiol- or amino-reactive label under mild conditions resulting in the labeling of said mutated kinase at the desired position in said region of proximity to the interface. Mild conditions refer to buffer pH (e.g. around pH=7 for thiol-reactive probes), ratio of label to kinase, temperature and length of the incubation step (for thiol-reactive probes e.g. 4 °C and overnight in the dark) which are known to the skilled person and provided with instruction manuals of providers of thiol- and amino-reactive probes. Such conditions may be fine-tuned in order to slow down the reaction of the chosen thiol- or amino-reactive label to ensure that labeling of said kinase is specific to the desired labeling site. In the case of fluorophore labeling, it is preferred to carry out the incubation in the dark. Increased light exposure may result in bleaching of the fluorophore and a less intense fluorescence emission. After labeling, the labeled kinase is preferably concentrated, purified by gel filtration, or washed several times with buffer to remove excess unreacted label. The wash buffer is typically the buffer used to store the labeled kinase and may also be the buffer in which the desired measurements are made.

In a preferred embodiment of the method of generating a kinase according to the invention, said amino acid residues according to step (a), the labeling of which is not desirable, are solvent-exposed. In this preferred embodiment, only those reactive amino acids are changed which are likely to react with fluorophores, spin labels or isotope labels containing a reactive group, given their exposure to solvent.

In a preferred embodiment the method of generating a kinase according to the invention further comprises (d) contacting the polypeptide obtained by the method of claim 15 with a kinase inhibitor; and (e) recording the fluorescence emission signal, the EPR signal or the NMR signal of the label of said polypeptide prior to and after step (d); wherein a difference in the two recordings is indicative of said polypeptide being suitable for the screening of kinase inhibitors.

The figures show:

**Figure 1:** a) Crystal structure of the full-length Akt protein. The PH domain is tightly associated with the kinase domain, forming a binding pocket for allosteric inhibitors (spheres) at the domain interface, approximately 15 Å from the ATP binding pocket (circled). A selection of known Akt inhibitors are shown. GSK690693 (XX14) and CCT128930 (XX15): commercially available ATP-competitive inhibitors. XX11: The first allosteric inter-domain inhibitor, found in a high-throughput campaign. MK-2206 (XX12): allosteric inhibitor currently in phase II clinical trials. Akti-1/2 (XX13): first allosteric inhibitor co-crystallized with full-length Akt (PDB code 3096) (Wu, W.-I. et al. Crystal Structure of Human AKT1 with an Allosteric Inhibitor Reveals a New Mode of Kinase Inhibition. PLoS One 5, e12913 (2010)).

XX10: second allosteric inhibitor co-crystallized with full-length Akt (PDB code 4EJN) (Ashwell, M.A. et al. Discovery and Optimization of a Series of 3-(3-Phenyl-3H-imidazo[4,5-b]pyridin-2-yl)pyridin-2-amines: Orally Bioavailable, Selective, and Potent ATP-Independent Akt Inhibitors. J. Med. Chem. 55, 5291-5310 (2012)). b) Schematic representation of the Akt regulation mechanism. Akt exists in an equilibrium between an inactive conformation, in which the PH domain binds to the kinase domain and obstructs access to the ATP binding pocket, and an active open conformation. Inter-domain allosteric inhibitors (oval) bind into a hydrophobic pocket formed by residues of the PH domain - kinase domain interface, locking Akt in the closed conformation. c) Schematic assay principle: PH domain labeled covalently with reporter fluorophore *via* synthetic cysteine (E49C). Transitions from open to closed conformations change the environment around the reporter fluorophore and therefore its fluorescence characteristics (dark and bright star, respectively).

**Figure 2:** Results of the iFLiK assay. a) Allosteric inhibitors (MK-2206 shown here) cause a bathochromic shift of the PyMPO emission spectrum (maximum from 560 nm to 576 nm), whereas ATP-competitive inhibitors like GSK690693 show no effect. b) Using the ratio of fluorescence intensities at 638 nm and 508 nm as the assay readout, the emission shift can be quantified. Again, the ATP-competitive GSK690693 has no effect. c) Structures of synthesized allosteric probe inhibitors XXa-g. d) Testing of the biological effect of Akt inhibitors. *In vitro* $IC_{50}$s were determined with the KinEASE assay against full-length Akt1 and ΔPH-Akt1. Cellular $GI_{50}$s were determined with the CellTiter Glo assay against BT474 and H441 cell lines. $K_d$s were determined with the iFLiK assay. All values are expressed in μM. n.i. = not inhibiting up to 200 μM. n.e. = not effective up to 30 μM. n.r. = no response up to 500 μM. n.m. = not measured.

**Figure 3:** Novel allosteric Akt inhibitors with pyrrolopyrimidine scaffold found in a high-throughput screen. a) Structures of compounds that showed an effect in activity and binding assays **(XX7a-h).** b) The two main binding modes for the pyrrolopyrimidines (**XX7a** shown as an example in sticks) as calculated by Glide. For reference in binding mode A (left) and B (right), the co-crystallized inhibitors Akti-1/2 (dark sticks) from 3096 and **XX10** (bright sticks) from 4EJN are displayed, respectively.

The following examples illustrate the invention.

**Example 1**:

Materials and methods

*iFLiK Assay Development and Validation*

Measurements were carried out in black 384-well plates (Greiner Bio One). Labeled Akt1 was diluted to 200 nM in the measurement buffer consisting of 50 mM HEPES pH 7.4, 200 mM NaCl and 0.01% Triton-X 100. Dilution series of candidate compounds were prepared in DMSO at 21X the final desired concentration. Compounds (0.5 μL) were then mixed with labeled Akt1 (10 μL) in triplicates and with measurement buffer only in quadruplicates at 12 different concentrations to achieve final compound concentrations ranging from 0.8 nM to 500 μM. Each well contained 5% v/v DMSO after mixing. Plates were then covered with an adhesive plastic foil and incubated for 2 h at RT prior to measurement of emission intensities at fluorophore-specific wavelengths (Table 1) using a Tecan Infinite M1000 plate reader. Compound auto-fluorescence was compensated by subtraction of the average background signals obtained in the absence of labeled kinase. The respective assay readout for the various Akt-fluorophore conjugates specified in Table 1 were fit to a Hill 4-parameter equation to determine $K_d$ values for allosteric Akt ligands. Each reaction was performed in triplicate and at least three independent measurements of each $K_d$ were determined for each ligand.

**Table 1:** Overview of fluorophore probes. Structures of reporter fluorophores tested for iFLiK, the observed effects when treated with MK-2206, the chosen assay readouts, Z'-factors, signal-to-noise ratios and determined $K_d$s for reference inhibitor MK-2206 and XX6b in brackets (n.m. = not measurable due to compound auto-fluorescence).

| Fluorophore | Excitation | Effect | Readout | Z' | S/N | $K_d$ MK-2206 (XX6b) |
|---|---|---|---|---|---|---|
| Acrylodan | 386 nm | 53 nm red-shift | 520/456 nm | 0.95 | 2.04 | n. m. (207 nM) |
| | | 0.5x signal decrease | 456 nm | 0.69 | 1.84 | n. m. (57 nM) |
| PyMPO | 419 nm | 16 nm red-shift | 638/508 nm | 0.90 | 1.92 | 96 nM (146nM) |

(continued)

| Fluorophore | Excitation | Effect | Readout | Z' | S/N | $K_d$ MK-2206 (XX6b) |
|---|---|---|---|---|---|---|
| DY-647 | 640 nm | 2 nm blue-shift | 697/660 nm | 0.72 | 1.16 | 75 nM |
| | | 2.2x signal increase | 672 nm | 0.56 | 2.21 | 63 nM |
| IANBD | 484 nm | 5 nm red-shift | 596/514 nm | 0.38 | 1.11 | 85 nM |
| Texas Red | 586 nm | 1.3x signal increase | 616 nm | 0.15 | 1.25 | 70 nM |
| | | 4 nm red-shift | 641/606 nm | 0.42 | 1.06 | 22 nM |
| Atto 565 | 559 nm | 1.3x signal increase | 695 nm | 0.23 | 1.27 | 63 nM |
| Eosin | 519 nm | 1.2x signal increase | 551 nm | 0.36 | 1.16 | 62 nM |

*Screening with iFLiK*

Screening initiatives were carried out in black 384-well plates (Greiner Bio One). PyMPO-labeled Akt1 was diluted to 200 nM in the measurement buffer consisting of 50 mM HEPES pH 7.4, 200 mM Nacl and 0.01% Triton-X 100. Each candidate compound was prepared in DMSO at 210 μM. Compounds (0.5 μL) were then mixed each with labeled Akt1 (10 μL) and with measurement buffer only to achieve final compound concentrations of 10 μM. Each well contained 5% v/v DMSO after mixing. Plates were then covered with an adhesive plastic foil and incubated for 2 h at RT prior to measurement using a Tecan Infinite M1000 plate reader emission intensities at 508 nm and 638 nm, using both 419 nm and 440 nm for excitation. Compound auto-fluorescence was compensated by subtraction of the average background signals obtained in the absence of labeled kinase. Ratio of fluorescence intensities $fl_{638}/fl_{508}$ were calculated from the background-subtracted values. For hit picking criteria, see Table 2 below.

**Table 2:** Small molecule screening data for iFLiK screen of in-house library.

| Category | Parameter | Description |
|---|---|---|
| Assay | Type of assay | *in vitro* |
| | Target | Human Akt1 (UniProt entry P31749) |
| | Primary measurement | Detection of reporter fluorescence |
| | Key reagents | PyMPO labeled Akt1 |
| | Assay protocol | See Materials and Methods |
| | Additional comments | Avoid unnecessary exposure to light |
| Library | Library size | 10,680 compounds |
| | Library composition | In-house library of synthesized compounds and commercial libraries |
| | Source Additional comments | Group of Prof. Dr. Herbert Waldmann |
| Screen | Format | Black 384-well plate (Greiner BioOne) |
| | Concentration(s) tested | 10 μM, 5% DMSO |
| | Plate controls | MK-2206 (positive) and DMSO (negative) |
| | Reagent/compound dispensing system | Eppendorf multichannel pipette (10 μL, 100 μL and 300 μL) |
| | Detection instrument and software | Tecan Infinite M1000 |
| | Assay validation/QC | Z' = 0.90 |
| | Correction factors | Background subtraction of compounds in buffer |
| | Normalization Additional comments | MK-2206 as 100%, DMSO control as 0% |

(continued)

| Category | Parameter | Description |
|---|---|---|
| Post-HTS analysis | Hit criteria<br>Hit rate<br>Additional assay(s)<br>Confirmation of hit purity and structure<br>Additional comments | >25% of positive control<br>0.22%<br>Tested for inhibitory activity with KinEASE assay<br>Verified by analytical HPLC-MS and repurchase |

*NanoTemper Assay Preparation & Execution*

For the NanoTemper assay, 3 equivalents of Alexa Fluor 568 NHS ester were added to 50 μM of kinase in a buffer consisting of 50 mM HEPES pH 8.3, 200 mM NaCl and 10% glycerol. After incubating 1 h in the dark at 4 °C, the protein was passed over a Superdex 75 10/300 GL (GE Healthcare) into a storage buffer consisting of 50 mM HEPES pH 7.3, 200 mM NaCl and 10% glycerol to remove unreacted fluorophore. The conjugated protein was then concentrated to approximately 30 μM, frozen in liquid nitrogen and stored at -80 °C.

Inhibitor dilution series of 21X of the final desired concentration were prepared in DMSO and then mixed with labeled Akt1 at 100 nM in a buffer consisting of 50 mM HEPES pH 7.4, 200 mM NaCl and 0.05% Tween-20. All solutions were incubated for 2 h at RT before transferring into enhanced gradient standard treated NanoTemper capillaries for measurement on a NanoTemper Monolith NT.115 using green excitation and emission filters. Capillaries were illuminated at 25 °C with 90% LED power for 5 s. Thermophoresis was tracked for 45 s at 100% laser intensity, back-diffusion for 5 s. Fluorescence intensities were measured at 8-9 s and 49-50 s, and fluorescence intensity ratios of hot/cold were used as assay readout. $K_d$s were determined using the internal evaluation software.

**Example 2:**

<u>Results</u>

*Assay Design & Development*

For our novel binding assay, we set out to use environmentally sensitive fluorophores to detect Akt changing from the active open to the inactive closed conformation (Figure 1c). In order to achieve the highest possible sensitivity, the reporter fluorophore has to be introduced close to the domain interface without disrupting inter-domain interactions. At the time, no crystal structure of the full-length Akt had been published. Analysing calculations and mutagenesis studies by Calleja *et al.* on the interaction between PH and kinase domain, however, we proposed Glu49 as suitable labeling position (Calleja, V. et al. Intramolecular and Intermolecular Interactions of Protein Kinase B Define Its Activation In Vivo. PLoS Biology 5, 780-791 (2007)). The subsequently published X-ray crystal structures (PDB entries 3096 and 4EJN) confirmed that Glu49 is sufficiently close to the domain interface (Ashwell, M.A. et al. Discovery and Optimization of a Series of 3-(3-Phenyl-3H-imidazo[4,5-b]pyridin-2-yl)pyridin-2-amines: Orally Bioavailable, Selective, and Potent ATP-Independent Akt Inhibitors. J. Med. Chem. 55, 5291-5310 (2012)). For a selective labeling of the reporter fluorophore to the desired position, we introduced an E49C mutation into Akt1 and removed all surface-exposed cysteines (C296S, C310S, C344S). This construct was introduced into a baculovirus system for expression in stably transfected TriEx Sf9 cells. Purification with nickel affinity and size exclusion chromatography yielded the purified protein, which was subsequently labeled with the reporter fluorophore. Tryptic digests with subsequent MS/MS analysis confirmed the successful and selective labeling at Cys49.

We synthesized a library of inhibitors (XX6a-g, Figure 2c). Selectivity in *in vitro* and cellular activity tests (Figure 2d) confirmed their allosteric mode of action. A collection of cysteine-reactive fluorophores used to label the purified protein and tested in combination with the clinical candidate MK-2206 as an allosteric reference inhibitor (positive detection control) and GSK690693 as an ATP-competitive reference inhibitor (negative detection control). The fluorophores were excited at their respective excitation maxima, which were determined in prior experiments. Some fluorophores like PyMPO or IANBD exhibited a shift in the emission spectrum and therefore in their fluorescence maximum, whereas an overall intensity increase could be observed with Atto565 or Eosin. Depending on the type of response observed, different assay readouts were used to determine ligand binding affinities. In the case of an intensity change, the fluorescence intensity at the emission maximum was used as the readout. In the case of spectrum shifting, the two wavelengths in the emission spectrum with half-maximal emission intensities were recorded, and the ratio of their emission intensities

served as the assay readout. By comparing the fluorescence of the kinase incubated with 5 μM MK-2206 to the apo kinase (n = 6), signal-to-noise ratios and Z'-factors were calculated for each fluorophore-labeled kinase to characterize the assay quality and reproducibility (Z' between 0.5 and 1 is generally considered ideal for high-throughput screening) (Zhang, J.-H., Chung, T.D.Y. & Oldenburg, K.R. A Simple Statistical Parameter for Use in Evaluation and Validation of High Throughput Screening Assays. J. Biomol. Screen. 4, 67-73 (1999)). Additionally, dose-response experiments were performed by incubating the labeled kinases with a dilution series of either MK-2206 or GSK690693 with final concentrations ranging between 0.8 nM and 50 μM. By plotting the respective assay response against the logarithmic inhibitor concentration, the $K_d$ was determined by fitting the data to a 4-point Hill function. Except for acrylodan, all fluorophores were able to detect MK-2206 binding to Akt with comparable $K_d$s but with varying degrees of reliability. The response appeared to be specific to allosteric inhibitors since the labeled kinase was unresponsive to the addition of the ATP-competitive inhibitor GSK690693. For acrylodan, high levels of MK-2206 auto-fluorescence at the acrylodan excitation wavelength rendered the measurement with this reference inhibitor impossible. Therefore, experiments were repeated for acrylodan- and PyMPO-labeled Akt using the less fluorescent **XX6b,** which revealed that acrylodan is also able to report on the conformational changes of Akt induced by allosteric inhibitors. For PyMPO, the assay response with **XX6b** was the same as with MK-2206. In summary, acrylodan and PyMPO proved to be the best performing probes for the iFLiK assay in terms of reliability. However, in order to avoid inhibitor auto-fluorescence, PyMPO was ultimately chosen for future experiments due to its excitation at longer wavelengths.

After extensive and careful assay optimisation, allosteric Akt inhibitors MK-2206 and **XX6a-g** as well as ATP-competitive GSK690693 **(XX14)** and CCT128930 **(XX15)** were surveyed with the PyMPO-labeled iFLiK construct in 384-well plates. Intrinsic compound auto-fluorescence was accounted for by subtracting fluorescence intensities of the respective compound alone in buffer. While the classic ATP-competitive Akt inhibitors had no effect on PyMPO fluorescence, the allosteric inhibitors provoked a bathochromic shift of the emission spectrum up to 16 nm, which allowed the determination of their $K_d$s (Figure 2a-b). The SAR gained from the $K_d$s perfectly mirrors the $IC_{50}$s determined in the activity-based KinEASE assay. Mixtures of inhibitors with PyMPO-labeled glutathione or 2-mercaptoethanol displayed no such effect, confirming that the observed responses are specific to the conformational change of Akt, and not due to interaction with the fluorophores alone. As the binding pocket and labeling site are more than 20 Å apart, an interaction between the ligand and the fluorophore (e.g. *via* resonance energy transfer or direct contact) is highly unlikely. This confirms that the observed spectral shifts are indeed due to changes in the fluorophore environment caused by inhibitor binding. This successfully demonstrates the ability of iFLiK to detect conformational changes of Akt induced by allosteric inhibitors. ATP-competitive inhibitors do not facilitate the closed conformation and, therefore, are not detected by the iFLiK assay.

In order to validate the assay results obtained with iFLiK, we employed the NanoTemper technology as an orthogonal assay, which measures changes in the diffusion kinetics of proteins upon ligand binding in a temperature gradient due to microscale thermophoresis (Wienken, C.J., Baaske, P., Rothbauer, U., Braun, D. & Duhr, S. Protein-binding assays in biological liquids using microscale thermophoresis (Nature communications 1, 100, doi:10.1038/ncomms1093 (2010)). Akt was labeled unspecifically *via* amines with Alexa Fluor 568 NHS-ester to enable tracking of kinase diffusion. In addition, AF568-labeled kinase was further labeled with the cysteine-reactive PyMPO maleimide. Both proteins preparations were individually measured in the NanoTemper assay with dilution series of MK-2206, **XX6a-g** and GSK690693. The appropriate filters where chosen to observe AF568 fluorescence only. In the temperature gradient induced by the IR laser, Akt pre-incubated with inhibitors depleted more quickly from the heat source than the DMSO control. The residual fluorescence intensity after 45 s was used as an indicator for the diffusion velocity, and the ratio of intensities at 9 s and 45 s was used as assay readout. $K_d$s were determined with the internal evaluation software based on a 4-point Hill equation and are comparable to the values obtained with iFLiK. The only exceptions are the weak inhibitors **XX6e** and **XX6g,** whose determined $K_d$s differ by about 2-4 fold. In contrast to iFLiK, NanoTemper was not able to distinguish between allosteric and ATP-competitive inhibitors since it detects binding regardless of the binding conformation of Akt. Interestingly, binding affinities for the Alexa Fluor 568-labeled Akt do not differ significantly differ from the dually-labeled AF568-PyMPO protein. This suggests that PyMPO labeling of the C49 does not have a significant impact on the inhibitor binding affinity. In contrast, mixtures of inhibitors with Alexa Fluor 568 alone (no kinase) displayed no thermophoretic effect. These findings confirm that iFLiK as a valid assay technology to determine binding affinities for allosteric Akt inhibitors.

*Screening of In-House Library*

With the iFLiK assay established and validated, we subsequently screened our in-house library of over 10,000 compounds, consisting of both commercially purchased as well as compounds synthesized in our group for various projects. While recording fluorescence intensities at 508 nm ($fl_{508}$) and 638 nm ($fl_{638}$) to detect a shift of the emission spectrum, two different wavelengths (419 nm and 440 nm) were chosen for excitation of the fluorophore: In order to avoid intrinsic compound fluorescence, which is more likely at shorter wavelengths, irradiation at 75% of the excitation

maximum (440 nm in the case of PyMPO) was considered a good compromise of signal intensity and interference by auto-fluorescence. However, compounds that do still fluoresce at 440 nm are more likely to emit at 508 nm due to their smaller Stokes shift than PyMPO. In this case, excitation at 419 nm will provide the signal with less background fluorescence.

In this 1-point screen at 10 μM, the 24 compounds that displayed >25% binding relative to MK-2206 as 100% positive control were considered hits and picked for follow-up. In these triplicate dose-response measurements, 13 out of 24 were validated to produce a dose-dependent change in the $fl_{508}/fl_{638}$ ratio. While **XX7a-c** evoked a bathochromic shift of the PyMPO fluorescence spectrum and **XX9** a hypsochromic shift, compounds like **XX8** also caused a reduction of the fluorescence intensity. In activity-based assays (KinEASE by Cisbio), 12 of the 13 validated hits from the screen indeed had an inhibitory effect on Akt. However, the 9 compounds that quenched the PyMPO fluorescence in the iFLiK experiments showed inhibition of both full length Akt1 and ΔPH-Akt1, whereas only the 3 compounds that solely provoked a bathochromic emission spectrum shift were selective for the full-length kinase, as would be expected for allosteric inhibitors.

XX8 and XX9 have the following structures:

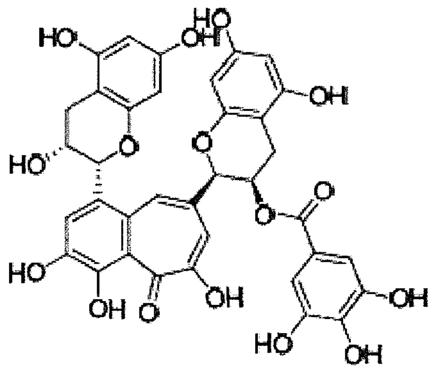

**XX8**

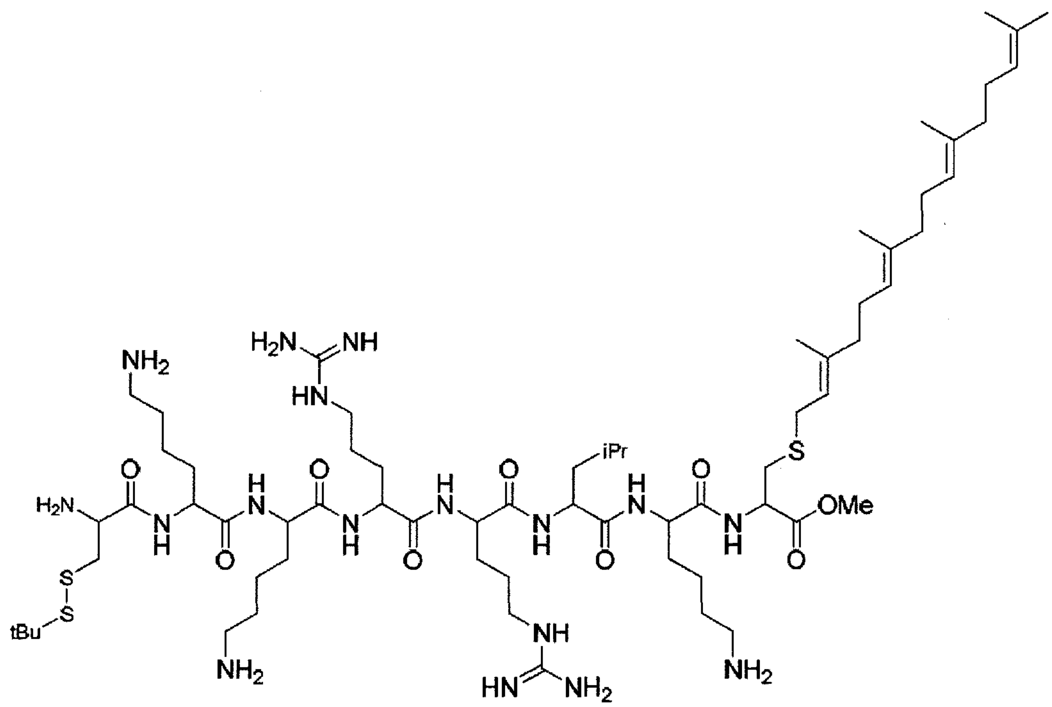

**XX9**

In order to understand the observed SAR, binding modes of these compounds were calculated by docking these structures into published full length crystal structures of Akt (PDB codes 3096 and 4EJN) (Glide, version 5.6, Schrödinger, Inc., New York, NY, 2010). Two main binding modes were calculated by Glide from both crystal structures

(Figure 3b). To further understand the binding of these compounds, we ordered and tested another 100 compounds based on this pyrrolopyrimidine scaffold to further explore the SAR (Figure 3a). These compounds also showed an effect in the iFLiK assay and exhibited selective inhibitory activity on the full-length Akt1, displaying a wide range of $K_d$s determined with iFLiK and $IC_{50}$s determined with KinEASE (Table 3).

**Table 3:** Results of pyrrolopyrimidine inhibitors in binding (iFLiK) and activity (KinEASE) assays. n.i. = no inhibition up to 200 μM. n.r. = no response up to 500 μM.

| | $K_d$ | $IC_{50}$ Akt1 | $IC_{50}$ ΔPH-Akt1 |
|---|---|---|---|
| **XX7a** | 2.2 ± 0.5 μM | 196 ± 102 μM | n.i. |
| **XX7b** | 7.8 ± 0.7 μM | 160 ± 44 μM | n.i. |
| **XX7c** | 20 ± 7μM | n.i. | n.i. |
| **XX7d** | 2.2 ± 0.7 μM | 71 ± 30 μM | n.i. |
| **XX7e** | 99 ± 61 μM | n.i. | n.i. |
| **XX7f** | 8 + 5 μM | 2760 ± 4431 μM | n.i. |
| **XX7g** | 14 ± 3 μM | 336 ± 111 μM | n.i. |
| **XX7h** | 264 ± 284 μM | 1435 ± 132 μM | n.i. |

## Claims

**1.** A polypeptide comprising or consisting of a protein kinase domain (KD) and one or more second domain(s), said second domain(s) binding to said KD and thereby modulating the activity of said KD, and/or said second domain(s) being selected from

(a) a pleckstrin homology domain (PH domain), said KD in that case being selected from Akt, PDK1, PKCμ, BTK, ITK, GRK2, Tec, ETK, TXK and ROCK;
(b) an SH2 domain, said KD in that case being selected from Abl, Src, BLK, BMX, BTK, Fer, Fes, FRK, FYN, HCK, JAK2, ITK, Lck, Lyn, MATK, BRK, Syk, Tec and TXK;
(c) an SH3 domain, said KD in that case being selected from Abl, Src, RSK and Lck;
(d) a protein kinase autoinhibition domain, said KD in that case being selected from AMPK, PAK1 and PKG;
(e) a protein kinase hydrophobic motif, said KD in that case being selected from Akt, PDK1, PKA and S6K;
(f) a C1 domain, said KD in that case being selected from PKC (including isoforms α, $\beta_I$, $\beta_{II}$, γ, δ, ε, η, θ, I, ζ, λ), ARAF, BRAF, MRCK, CIT, ROCK1 and ROCK2; and
(g) a C2 domain, said KD in that case being selected from PKCα, PKC$\beta_I$, PKC$\beta_{II}$ and PKCγ;

said polypeptide containing,

(1) if one second domain is present, within a distance of not more than 15 Å and not less than 1 Å from the interface between said KD and said second domain, exactly one or exactly two environmentally sensitive labels, or,
(2) if more than one second domain is present, within a distance of not more than 15 Å and not less than 1 Å from each of one, more or all of the interfaces between said KD and said second domains, respectively, exactly one or exactly two environmentally sensitive labels,

wherein said label(s) is/are (a) fluorophore(s), (a) spin label(s) or (an) isotope label(s),

(i) the fluorophore or said spin label is covalently bound to a thiol group or side chain amino group of an amino acid residue,
(ii) the isotope label is a moiety comprising one or more atoms which are isotopes with non-zero nuclear spin, said moiety being covalently bound to a thiol group or side chain amino group of an amino acid residue, or the isotope label is an amino acid residue, said amino acid residue comprising one or more atoms which are isotopes with non-zero nuclear spin, wherein said isotopes with non-zero nuclear spin are enriched as compared to the natural occurrence thereof, wherein, in case exactly two labels are present for a given interface, a first label is present on said KD and a second label is present on the second domain forming said given interface.

**2.** The polypeptide of claim 1, wherein

(a) exactly one second domain is present; and/or
(b) for each one of one, more or all of the interfaces exactly one label is present within a distance as defined in claim 1, and wherein the second domain(s) carries/carry said exactly one label.

**3.** The polypeptide of claim 1 or 2, wherein said polypeptide is a kinase of the Akt family, preferably Akt1, or a fragment thereof, said fragment comprising said KD and a PH domain.

**4.** The polypeptide of any one of the preceding claims, wherein said fluorophore is selected from Acrylodan, PyMPO, DY-647, IANBD, IAEDANS, IAANS, Texas Red, Atto 565 and Eosin.

**5.** The polypeptide of any one of the preceding claims, wherein said label is the only fluorophore, spin label or isotope label present in said polypeptide.

**6.** The polypeptide of any one of the preceding claims, wherein the amino acid sequence of said polypeptide is

(a) the amino acid sequence of the wild type form of said polypeptide or of a fragment thereof, said fragment comprising said KD and said second domain; or
(b)

(i) differs from the amino acid sequence of said wild type form or fragment thereof by the presence of one amino acid residue containing a thiol group or side chain amino group at a position where the amino acid sequence of said wild type form does not contain such a residue, said residue being within a distance from said interface as defined in claim 1; and/or
(ii) differs from the amino acid sequence of said wild type form or fragment thereof by the absence of one or more amino acid residues containing a thiol group or side chain amino group,
provided that exactly one amino acid residue, preferably exactly one solvent-exposed amino acid residue containing a thiol group or side chain amino group is present in said polypeptide within a distance from said interface as defined in claim 1.

**7.** A method of screening for ligands of a second polypeptide, said second polypeptide being

(i) identical to the polypeptide as defined in any one of claims 1 to 6;
(ii) the wild type form of said polypeptide as defined in any one of claims 1 to 6; or
(iii) a fragment of the polypeptide of (i) or (ii), said fragment comprising the KD and the second domain as comprised in the polypeptide of (i);

said method comprising:

(a) contacting the polypeptide of any one of claims 1 to 6 with a candidate compound; and
(b) recording the fluorescence emission signal, the EPR signal or the NMR signal of said label defined in any one of claims 1 to 6,
wherein a difference in signal as compared to the signal in absence of said candidate compound is indicative of said candidate compound being a ligand of said second polypeptide.

**8.** A method of screening for inhibitors of kinase activity of a second polypeptide said second polypeptide being

(i) identical to the polypeptide as defined in any one of claims 1 to 6;
(ii) the wild type form of said polypeptide as defined in any one of claims 1 to 6; or
(iii) a fragment of the polypeptide of (i) or (ii), said fragment comprising the KD and the second domain as comprised in the polypeptide of (i);

said method comprising the method of claim 7 and

(c) determining the kinase activity of the KD comprised in the second polypeptide in the presence of said candidate compound,
wherein a decrease in activity as compared to the absence of said candidate compound is indicative of said candidate compound being an inhibitor of said kinase activity.

**9.** A compound of the following formula (I)

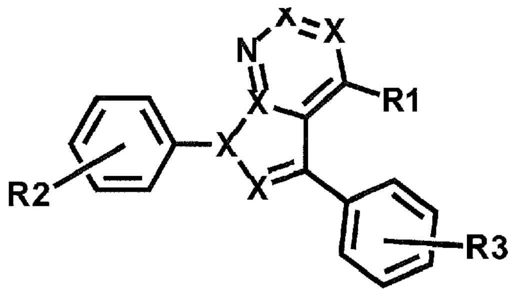

wherein
X is C, N, O or S, any free valence of C, N, O or S being filled with hydrogen;
$R^1$ is arylalkyl-Y, heteroarylalkyl-Y, aryl-Y, heteroaryl-Y, arylalkenyl-Y or heteroarylalkenyl-Y; aryl and heteroaryl being 5- or 6-membered rings, heteroatoms in said heteroaryl being selected from S, N and O, the number of said heteroatoms being 1, 2 or 3, alkyl being $C_1$ to $C_4$ alkyl, alkenyl being $C_1$ to $C_4$ alkenyl,
Y being selected from $CH_2$, NH, SH and O;
$R^2$ and $R^3$ are independently either absent or selected from halogen and $C_1$ to $C_4$ alkyl wherein the number of occurrences of $R^2$ and $R^3$ is independently selected from 1,2 and 3.

**10.** A pharmaceutical composition comprising a compound as defined in claim 9.

**11.** A compound as defined in claim 9 for use in a method of treating cancer.

**12.** A method of quantifying ligand or inhibitor binding to a polypeptide comprising a KD and a second domain, said second domain being as defined in claim 1, said method comprising

(a)

(i) contacting a polypeptide according to any one of claims 1 to 6 with different concentrations of a ligand or inhibitor; and/or
(ii) contacting a complex, the complex comprising or consisting of the polypeptide according to any one of claims 1 to 6 and a ligand or inhibitor, with different concentrations of a second polypeptide as defined in claim 7;

(b) recording the signal emitted by the label of said polypeptide according to any one of claims 1 to 6 at a given time point after said contacting and for each concentration of ligand or inhibitor according to (a)(i) and/or for each concentration of said second polypeptide according to (a)(ii); and
(c) determining the dissociation and/or association constant of said complex from the concentration dependence of said signal recorded according to (b).

**13.** A method of quantifying ligand or inhibitor binding to a kinase, said method comprising

(a)

(i) contacting a polypeptide according to any one of claims 1 to 6 with at least one concentration of a ligand or inhibitor; and/or
(ii) contacting a complex, the complex comprising or consisting of the polypeptide according to any one of claims 1 to 6 and a ligand or inhibitor, with at least one concentration of a second single polypeptide as defined in claim 7;

(b) recording the signal emitted by the label defined in any one of claims 1 to 6 as a function of time upon said contacting according to (a); and
(c)

(i) determining the rate constant governing the time dependence of said signal according to (b) for (each

of) the concentration(s) according to (a)(i) and determining $k_{on}$ therefrom; and/or
(ii) determining the rate constant governing the time dependence of said signal according to (b) for (each of) the concentration(s) according to (a)(ii) and determining $k_{off}$ therefrom.

**14.** A method of generating a polypeptide suitable for the screening of kinase inhibitors, said method comprising

(a) deleting one or more amino acid residues having a free thiol group or free side chain amino group, if any, present in a polypeptide comprising a KD and a second domain, said second domain being defined as in claim 1, the labeling of said amino acid residues not being desirable, or replacing them with amino acid residues which do not contain a free thiol or side chain amino group, wherein preferably said amino acid residues the labeling of which is not desirable are solvent-exposed;
(b) replacing in said polypeptide according to (a) an amino acid residue within a distance from the interface as defined in claim 1 with an amino acid residue containing a free thiol or side chain amino group if no amino acid residue containing a free thiol or side chain amino group is or would be present within said distance after step (a), wherein steps (a) and (b) can be effected in any order; and
(c) labeling the polypeptide obtained from steps (a) and (b) with a thiol- or amino-reactive fluorophore, thiol- or amino-reactive spin label, and/or thiol- or amino-reactive isotope label, thereby obtaining said polypeptide suitable for the screening of kinase inhibitors.

**15.** The method of claim 14, further comprising

(d) contacting the polypeptide obtained by the method of claim 15 with a kinase inhibitor; and
(e) recording the fluorescence emission signal, the EPR signal or the NMR signal of the label of said polypeptide prior to and after step (d);
wherein a difference in the two recordings is indicative of said polypeptide being suitable for the screening of kinase inhibitors.

Figure 1

a

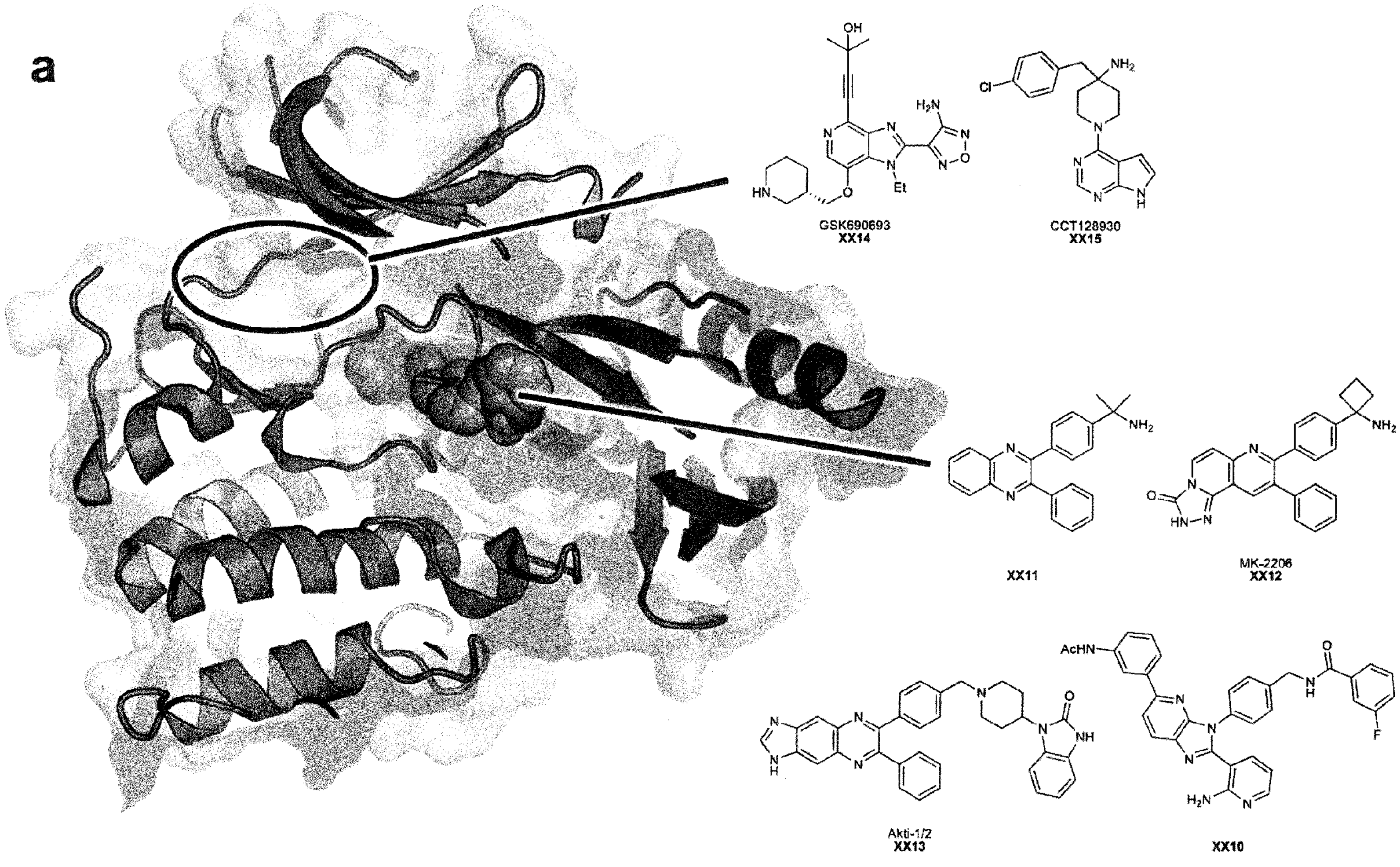

GSK690693
**XX14**

CCT128930
**XX15**

**XX11**

MK-2206
**XX12**

Akti-1/2
**XX13**

**XX10**

Figure 1 continued

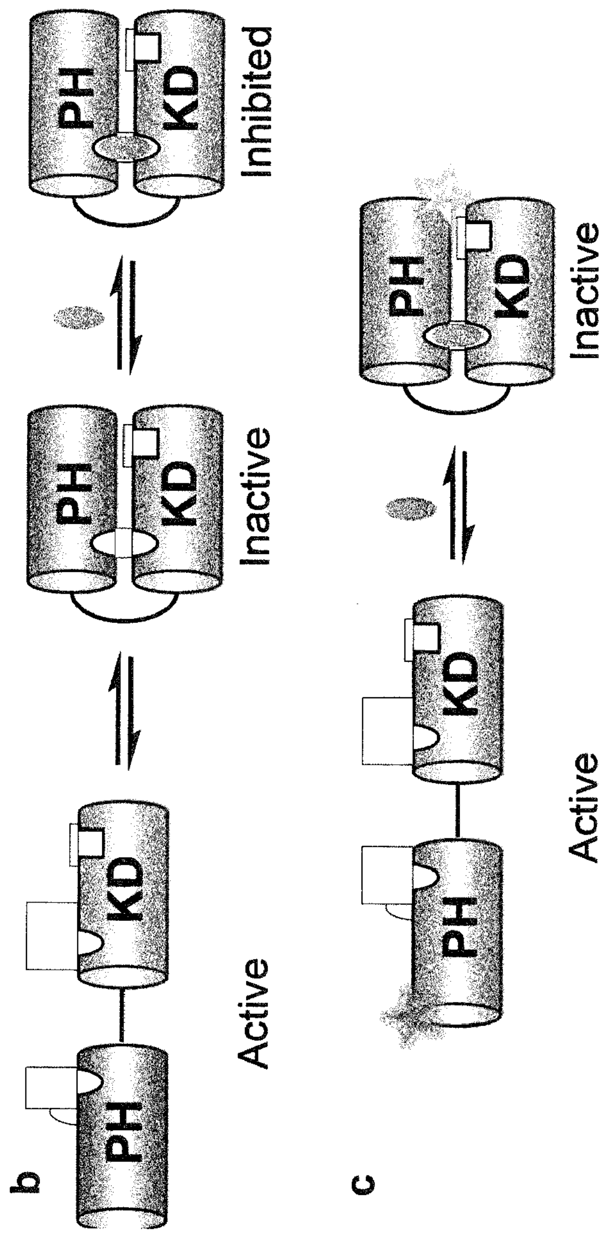

## Figure 2

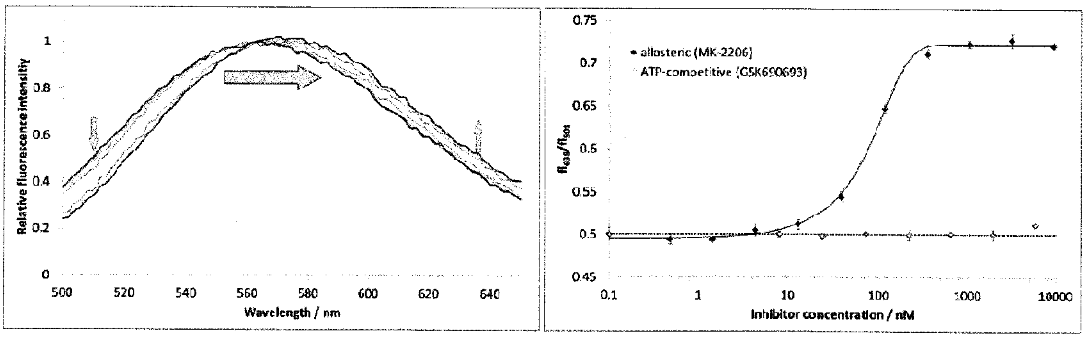


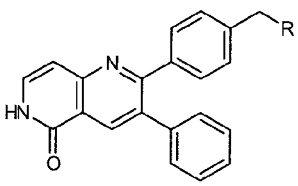
XX6a-g

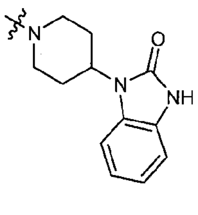
a

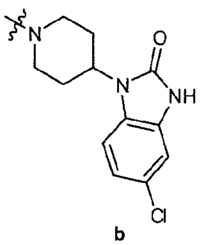
b

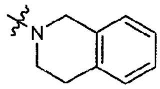
c

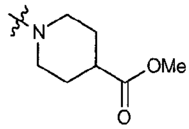
d

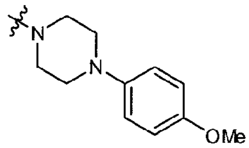
e

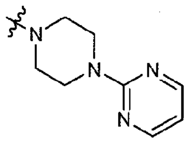
f

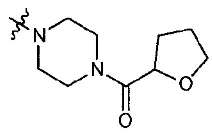
g

| | $IC_{50}$ Akt1 | $IC_{50}$ ΔPH-Akt1 | $GI_{50}$ BT474 | $GI_{50}$ H441 | $K_d$ iFLiK |
|---|---|---|---|---|---|
| **XX6a** | 0.03 ± 0.005 | n.i. | 7 ± 2 | n.e. | 0.41 ± 0.07 |
| **XX6b** | 0.01 ± 0.004 | n.i. | 3.2 ± 0.6 | n.e. | 0.15 ± 0.04 |
| **XX6c** | 0.9 ± 0.2 | n.i. | 17 ± 2 | n.e. | 6 ± 2 |
| **XX6d** | 0.6 ± 0.1 | n.i. | 18 ± 4 | n.e. | 0.9 ± 0.3 |
| **XX6e** | 1.4 ± 0.5 | n.i. | 19 ± 4 | n.e. | 7.2 ± 0.9 |
| **XX6f** | 0.4 ± 0.05 | n.i. | 13 ± 1 | n.e. | 1.1 ± 0.2 |
| **XX6g** | 2.1 ± 0.2 | n.i. | 19 ± 3 | n.e. | 8 ± 1 |
| MK-2206 | 0.006 ± 0.002 | n.i. | 0.3 ± 0.1 | n.e. | 0.085 ± 0.010 |
| GSK690603 | 0.0004 ± 0.0002 | 0.0007 ± 0.0004 | 0.7 ± 0.2 | n.e. | n.r. |
| CCT128930 | 0.029 ± 0.009 | 0.030 ± 0.007 | n.m. | n.m. | n.r. |

Figure 3

**a**

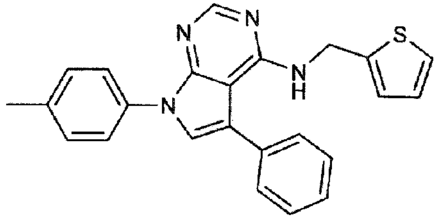

XX7a

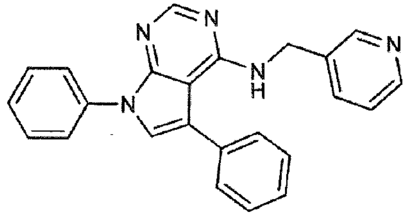

XX7b

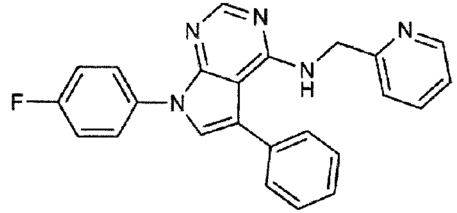

XX7c

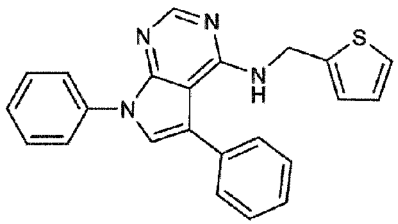

XX7d

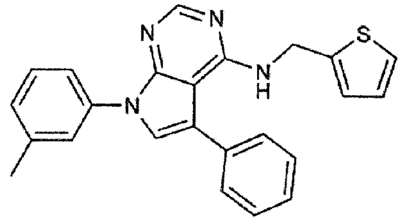

XX7e

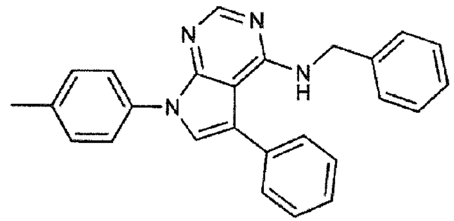

XX7f

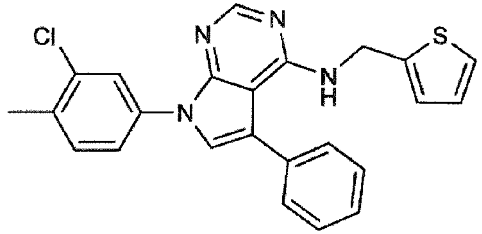

XX7h

**Figure 3** continued

**b**

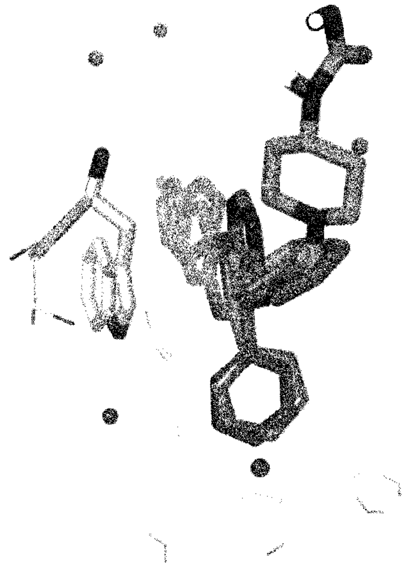

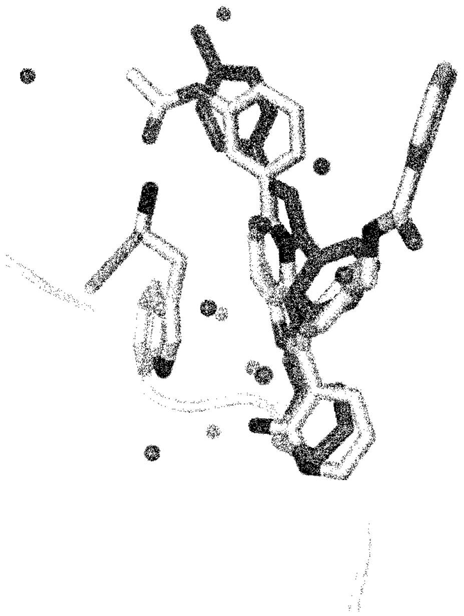

Europäisches Patentamt
European Patent Office
Office européen des brevets

# EUROPEAN SEARCH REPORT

Application Number
EP 13 17 0412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JEFFREY R SIMARD ET AL: "A new screening assay for allosteric inhibitors of cSrc", NATURE CHEMICAL BIOLOGY, vol. 5, no. 6, 1 June 2009 (2009-06-01), pages 394-396, XP055076451, ISSN: 1552-4450, DOI: 10.1038/nchembio.162 * Supplemental material; figures 1-3 * | 1,2,4-8, 12-15 | INV. A61K31/519 G01N33/533 G01N33/68 C07D239/94 |
| Y | WEN-I WU ET AL: "Crystal Structure of Human AKT1 with an Allosteric Inhibitor Reveals a New Mode of Kinase Inhibition", PLOS ONE, vol. 5, no. 9, 23 September 2010 (2010-09-23), page e12913, XP055076635, DOI: 10.1371/journal.pone.0012913 * figures * | 1-8, 12-15 | |
| Y | FLORIAN GREBIEN ET AL: "Targeting the SH2-Kinase Interface in Bcr-Abl Inhibits Leukemogenesis", CELL, CELL PRESS, US, vol. 147, no. 2, 31 August 2011 (2011-08-31), pages 306-319, XP028317886, ISSN: 0092-8674, DOI: 10.1016/J.CELL.2011.08.046 [retrieved on 2011-09-16] * figures * | 1-8, 12-15 | TECHNICAL FIELDS SEARCHED (IPC) A61K G01N C07D |

-/--

The present search report has been drawn up for all claims

2

| Place of search | Date of completion of the search | Examiner |
|---|---|---|
| Munich | 29 August 2013 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 0412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RALF SCHNEIDER ET AL: "Direct Binding Assay for the Detection of Type IV Allosteric Inhibitors of Abl", JOURNAL OF THE AMERICAN CHEMICAL SOCIETY, vol. 134, no. 22, 6 June 2012 (2012-06-06), pages 9138-9141, XP055076369, ISSN: 0002-7863, DOI: 10.1021/ja303858w * Suppl. material; figures 1-3 * ----- | 1,2,4-8, 12-15 | |
| Y | MARK A. ASHWELL ET AL: "Discovery and Optimization of a Series of 3-(3-Phenyl-3 H -imidazo[4,5- b ]pyridin-2-yl)pyridin-2-amines: Orally Bioavailable, Selective, and Potent ATP-Independent Akt Inhibitors", JOURNAL OF MEDICINAL CHEMISTRY, vol. 55, no. 11, 14 June 2012 (2012-06-14), pages 5291-5310, XP055076710, ISSN: 0022-2623, DOI: 10.1021/jm300276x * figures * ----- | 1-8, 12-15 | |
| | | | TECHNICAL FIELDS SEARCHED (IPC) |
| A | LORI KRIM GAVRIN ET AL: "Approaches to discover non-ATP site kinase inhibitors", MEDCHEMCOMM, vol. 4, no. 1, 1 January 2013 (2013-01-01), pages 41-51, XP055076726, ISSN: 2040-2503, DOI: 10.1039/c2md20180a * page 44 - page 45 * ----- -/-- | 1-15 | |

The present search report has been drawn up for all claims

2

| Place of search | Date of completion of the search | Examiner |
|---|---|---|
| Munich | 29 August 2013 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 0412

| Category | Citation of document with indication, where appropriate, of relevant passages | Relevant to claim | CLASSIFICATION OF THE APPLICATION (IPC) |
|---|---|---|---|
| X | ZHIZHOU FANG ET AL: "Strategies for the Selective Regulation of Kinases with Allosteric Modulators: Exploiting Exclusive Structural Features", ACS CHEMICAL BIOLOGY, vol. 8, no. 1, 18 January 2013 (2013-01-18), pages 58-70, XP055076454, ISSN: 1554-8929, DOI: 10.1021/cb300663j * figures 2,3; table 1 * ----- | 1-15 | |
| A | RALF SCHNEIDER ET AL: "Overcoming Compound Fluorescence in the FLiK Screening Assay with Red-Shifted Fluorophores", JOURNAL OF THE AMERICAN CHEMICAL SOCIETY, vol. 135, no. 22, 14 May 2013 (2013-05-14), pages 8400-8408, XP055076370, ISSN: 0002-7863, DOI: 10.1021/ja403074j * the whole document * ----- | 1-15 | TECHNICAL FIELDS SEARCHED (IPC) |
| X | WO 2010/009886 A1 (MAX PLANCK GESELLSCHAFT [DE]; RAUH DANIEL [DE]; SIMARD JEFFREY RAYMOND) 28 January 2010 (2010-01-28) * claims; examples * ----- | 1,2,4-8, 12-15 | |
| X | WO 2010/119138 A1 (MAX PLANCK GESELLSCHAFT [DE]; RAUH DANIEL [DE]; SIMARD JEFFREY RAYMOND) 21 October 2010 (2010-10-21) * claims; examples * ----- | 1,2,4-8, 12-15 | |
| X | WO 2012/130984 A1 (MAX PLANCK GESELLSCHAFT [DE]; RAUH DANIEL [DE]; SIMARD JEFFREY RAYMOND) 4 October 2012 (2012-10-04) * claims * ----- -/-- | 1,2,4-8, 12-15 | |

The present search report has been drawn up for all claims

2

| Place of search | Date of completion of the search | Examiner |
|---|---|---|
| Munich | 29 August 2013 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 0412

| Category | Citation of document with indication, where appropriate, of relevant passages | Relevant to claim | CLASSIFICATION OF THE APPLICATION (IPC) |
|---|---|---|---|
| X | BALAKIN KONSTANTIN V ET AL: "Property-based design of GPCR-targeted library", JOURNAL OF CHEMICAL INFORMATION AND COMPUTER SCIENCES, AMERICAN CHEMICAL SOCIETY, COLOMBUS,OHIO, US, vol. 42, no. 6, 1 November 2002 (2002-11-01), pages 1332-1342, XP002434883, ISSN: 0095-2338, DOI: 10.1021/CI025538Y * figure 5 * | 9,10 | |
| X | WO 2010/066629 A2 (HOFFMANN LA ROCHE [CH]; DILLON MICHAEL PATRICK [US]; DU BOIS DAISY JOE) 17 June 2010 (2010-06-17) * claims * | 9,10 | |
| X | DATABASE Chemcats [Online] Chemical Abstracts Service (CAS), USA.; 1 January 2013 (2013-01-01), Ambinter Stock Screening Collection: XP007922199, * the whole document * | 9 | TECHNICAL FIELDS SEARCHED (IPC) |

The present search report has been drawn up for all claims

2

| Place of search | Date of completion of the search | Examiner |
|---|---|---|
| Munich | 29 August 2013 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 17 0412

This annex lists the patent family members relating to the patent documents cited in the above-mentioned European search report. The members are as contained in the European Patent Office EDP file on
The European Patent Office is in no way liable for these particulars which are merely given for the purpose of information.

29-08-2013

| Patent document cited in search report | Publication date | Patent family member(s) | Publication date |
|---|---|---|---|
| WO 2010009886 A1 | 28-01-2010 | AU 2009273465 A1 | 28-01-2010 |
| | | CA 2731357 A1 | 28-01-2010 |
| | | EP 2326728 A1 | 01-06-2011 |
| | | JP 2011528560 A | 24-11-2011 |
| | | US 2011212475 A1 | 01-09-2011 |
| | | WO 2010009886 A1 | 28-01-2010 |
| WO 2010119138 A1 | 21-10-2010 | AU 2010238491 A1 | 10-11-2011 |
| | | CA 2758849 A1 | 21-10-2010 |
| | | EP 2241619 A1 | 20-10-2010 |
| | | EP 2419509 A1 | 22-02-2012 |
| | | JP 2013504302 A | 07-02-2013 |
| | | US 2012107836 A1 | 03-05-2012 |
| | | WO 2010119138 A1 | 21-10-2010 |
| WO 2012130984 A1 | 04-10-2012 | AU 2012234209 A1 | 22-08-2013 |
| | | EP 2505572 A1 | 03-10-2012 |
| | | WO 2012130984 A1 | 04-10-2012 |
| WO 2010066629 A2 | 17-06-2010 | US 2010144758 A1 | 10-06-2010 |
| | | WO 2010066629 A2 | 17-06-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3773919 A
- EP 58481 A
- EP 133988 A
- DE 3218121
- EP 52322 A
- EP 36676 A
- EP 88046 A
- EP 143949 A
- EP 142641 A
- JP 58118008 A
- US 4485045 A
- US 4544545 A
- EP 102324 A


### Non-patent literature cited in the description


- **RABILLER, M. et al.** Proteus in the world of proteins: conformational changes in protein kinases. *Arch. Pharm. (Weinheim, Ger.),* 2010, vol. 343, 193-206
- **BHAGWAT, S.S.** Kinase inhibitors for the treatment of inflammatory and autoimmune disorders. *Purinergic Signal,* 2009, vol. 5, 107-115
- **RAUCH, J. ; VOLINSKY, N. ; ROMANO, D. ; KOLCH, W.** The secret life of kinases: functions beyond catalysis. *Cell Commun. Signal.,* 2011, vol. 9, 23
- **HANKS, STEVEN K. ; HUNTER, TONY.** The eukaryotic protein kinase superfamily: kinase (catalytic) domain structure and classification. Academic Press, 1995, vol. 9, 576-596
- **RABILLER, M. ; GETLIK, M. ; KLÜTER, S. ; RICHTERS, A. ; TÜCKMANTEL, S. ; SIMARD, J.R. ; RAUH, D.** Proteus in the world of proteins: Conformational changes in protein kinases. *Arch. Pharm. Chem. Life Sci.,* 2010, vol. 343, 193-206
- **SEROTA TAYLOR, S. ; RADZIO-ANDZELM, ELZBIETA.** Three protein kinase structures define a common motif. *Structure,* 1994, vol. 2, 345-355
- **M. PUNTA ; P.C. COGGILL ; R.Y. EBERHARDT ; J. MISTRY ; J. TATE ; C. BOURSNELL ; N. PANG ; K. FORSLUND ; G. CERIC ; J. CLEMENTS.** *Nucleic Acids Research,* 2012, vol. 40, D290-D301
- **R. J. HASLAM ; H. B. KOIDE ; B. A. HEMMINGS.** Pleckstrin domain homology. *Nature,* 1993, vol. 363, 309-310
- **T. PAWSON.** SH2 and SH3 domains. *Curr. Opin. Struct. Biol.,* 1992, vol. 2, 432-437
- **T. PAWSON ; J. SCHLESSINGERT.** SH2 and SH3 domains. *Current Biology,* 1993, vol. 3, 434-442
- **B. J. MAYER ; D. BALTIMORE.** Signalling through SH2 and SH3 domains. *Trends Cell Biol,* 1993, vol. 3, 8-13
- **T. E. SMITHGALL.** SH2 and SH3 Domains: Potential Targets for Anti-Cancer Drug Design. *J. Pharmacol. Toxicol. Methods,* 1995, vol. 34, 125-132
- **L. CHEN ; Z.-H. JIAO ; L.-S. ZHENG ; Y.-Y. ZHANG ; S.-T. XIE ; Z.-X. WANG ; J.-W. WU.** *Structural insight into the autoinhibition mechanism of AMP-activated protein kinase,* 2009, vol. 459, 1146-1150
- **T. PANG ; B. XIONG ; J.-Y. LI ; B.-Y. QIU ; G.-Z. JIN ; J.-K. SHEN ; J. LI.** Conserved a-Helix Acts as Autoinhibitory Sequence in AMP-activated Protein Kinase a Subunits. *J. Biol. Chem.,* 2007, vol. 282, 495-506
- **M. LEI ; W. LU ; W. MENG ; M.-C. PARRINI ; M. J. ECK ; B. J. MAYER ; S. C. HARRISON.** Structure of PAK1 in an Autoinhibited Conformation Reveals a Multistage Activation Switch. *Cell,* 2000, vol. 102, 387-397
- **G. M. BOKOCH.** Biology of the p21-activated Kinases. *Annu. Rev. Biochem.,* 2003, vol. 72, 743-781
- **Z. M. JAFFER ; J. CHERNOFF.** p21-Activated kinases: three more join the Pak. *Int. J. Biochem. Cell Biol.,* 2002, vol. 34, 713-717
- **M. CARLA PARRINI ; M. LEI ; S. C. HARRISON ; B. J. MAYER.** Pak1 Kinase Homodimers Are Autoinhibited in trans and Dissociated upon Activation by Cdc42 and Rac1. *Mol. Cell,* 2002, vol. 9, 73-83
- **Y.-H. HSU ; D. A. JOHNSON ; J. A. TRAUGH.** Analysis of Conformational Changes during Activation of Protein Kinase Pak2 by Amide Hydrogen/Deuterium Exchange. *J. Biol. Chem.,* 2008, vol. 283, 36397-36405
- **S. H. FRANCIS ; J. D. CORBIN.** Structure and Function of Cyclic Nucleotide-dependent Protein Kinases. *Annu. Rev. Physiol.,* 1994, vol. 56, 237-272

- **F. HOFMANN ; D. BERNHARD ; R. LUKOWSKI ; P. WEINMEISTER.** cGMP regulated protein kinases (cGK. *Handb. Exp. Pharmacol.,* 2009, vol. 191, 137-162
- **B. W. OSBORNE ; J. WU ; C. J. MCFARLAND ; C. K. NICKL ; B. SANKARAN ; D. E. CASTEEL ; V. L. WOODS, JR. ; A. P. KORNEV ; S. S. TAYLOR ; W. R. DOSTMANN.** Crystal Structure of cGMP-Dependent Protein Kinase Reveals Novel Site of Interchain Communication. *Structure,* 2011, vol. 19, 1317-1327
- **R. A. ATKINSON ; V. SAUDEK ; J. P. HUGGINS ; J. T. PELTON.** H NMR and Circular Dichroism Studies of the N-Terminal Domain of Cyclic GMP Dependent Protein Kinase: A Leucine/isoleucine Zipper. *Biochemistry,* 1991, vol. 30, 9387-9395
- **N. KANNAN ; N. HASTE ; S. TAYLOR ; A. F. NEUWALD.** The hallmark of AGC kinase functional divergence is its C-terminal tail, a cis-acting regulatory module. *Proc. Natl. Acad. Sci.,* 2007, vol. 104, 1272-1277
- **A. C. NEWTON.** Regulation of the ABC kinases by phosphorylation: protein kinase C as a paradigm. *Biochem. J.,* 2003, vol. 370, 361-371
- **F. COLÓN-GONZÁLEZ ; M. G. KAZANIETZ.** C1 domains exposed: From diacylglycerol binding to protein-protein interactions. *Biochim. Biophys. Acta,* 2006, vol. 1761, 827-837
- **G. ZHANG ; M. G KAZANIETZ ; P. M. BLUMBERG ; J. H HURLEY.** Crystal structure of the Cys2 activator-binding domain of protein kinase Cδ in complex with phorbol ester. *Cell,* 1995, vol. 81, 917-924
- **A. AZZI ; D. BOSCOBOINIK ; C. HENSEY.** The protein kinase C family. *Eur. J. Biochem.,* 1992, vol. 208, 547-557
- **R. B. SUTTON ; B. A DAVLETOV ; A. M. BERGHUIS ; T. C. SUDHOF ; S. R SPRANG.** Structure of the first C2 domain of synaptotagmin I: A novel Ca2+/phospholipid-binding fold. *Cell,* 1995, vol. 80, 929-938
- **D. ZHANG ; L. ARAVIND.** Identification of novel families and classification of the C2 domain superfamily elucidate the origin and evolution of membrane targeting activities in eukaryotes. *Gene,* 2010, vol. 469, 18-30
- *Nucleic Acids Res.,* 2011, vol. 39, D214-D219
- **SIDMAN, U. et al.** *Biopolymers,* 1983, vol. 22, 547-556
- **R. LANGER et al.** *J. Biomed. Mater. Res.,* 1981, vol. 15, 167-277
- **R. LANGER.** *Chem. Tech.,* 1982, vol. 12, 98-105
- **EPSTEIN et al.** *Proc. Natl. Acad. Sci. (USA,* 1985, vol. 82, 3688-3692
- **HWANG et al.** *Proc. Natl. Acad. Sci. (USA,* 1980, vol. 77, 4030-4034
- **WU, W.-I. et al.** Crystal Structure of Human AKT1 with an Allosteric Inhibitor Reveals a New Mode of Kinase Inhibition. *PLoS One,* 2010, vol. 5, e12913
- **ASHWELL, M.A. et al.** Discovery and Optimization of a Series of 3-(3-Phenyl-3H-imidazo[4,5-b]pyridin-2-yl)pyridin-2-amines: Orally Bioavailable, Selective, and Potent ATP-Independent Akt Inhibitors. *J. Med. Chem.,* 2012, vol. 55, 5291-5310
- **CALLEJA, V. et al.** Intramolecular and Intermolecular Interactions of Protein Kinase B Define Its Activation In Vivo. *PLoS Biology,* 2007, vol. 5, 780-791
- **ZHANG, J.-H. ; CHUNG, T.D.Y. ; OLDENBURG, K.R.** A Simple Statistical Parameter for Use in Evaluation and Validation of High Throughput Screening Assays. *J. Biomol. Screen.,* 1999, vol. 4, 67-73
- **WIENKEN, C.J. ; BAASKE, P. ; ROTHBAUER, U. ; BRAUN, D. ; DUHR, S.** Protein-binding assays in biological liquids using microscale thermophoresis. *Nature communications,* 2010, vol. 1, 100